# EUROPEAN PATENT APPLICATION

(11) **EP 3 753 579 A1**
(43) Date of publication of application: **23.12.2020**
(21) Application number: 19753873.9
(22) Date of filing: 31.01.2019
(51) Int. Cl.: A61K 47/68, A61K 38/07, A61P 35/00

(54) **ANTI-TRAILR2 ANTIBODY-TOXIN-CONJUGATE AND PHARMACEUTICAL USE THEREOF IN ANTI-TUMOR THERAPY**

(30) Priority: 13.02.2018 CN 201810150870; 13.02.2018 CN 201810150871
(71) Applicant: Yantai Obioadc Biomedical Technology Ltd., Yantai, Shandong 264006 (CN)
(72) Inventor: ZHENG, Dexian, Shanghai 201321 (CN); ZHANG, Shuyong, Shanghai 201321 (CN); PAN, Oudong, Shanghai 201321 (CN); ZHENG, Chao, Shanghai 201321 (CN); ZHU, Wan, Shanghai 201321 (CN); XIA, Qingmei, Shanghai 201321 (CN)
(74) Representative: Cabinet Laurent & Charras
(86) International application number: PCT/CN2019/074139
(87) International publication number: WO 2019/157973

(57) **Abstract**

Provided is a broad-spectrum, efficient and anti-tumor anti-TRAILR2 antibody-toxin-conjugate (ADC, named Zapadcine-1(a, b, c, d) and Zapadcine-3(a, b, c, d, e)). Toxin with cytotoxic effect is linked to an anti-TRAILR2 humanized monoclonal antibody by means of a covalent bond by using disulfide bond bridging or conventional coupling technology and chemical linkers, so as to form an anti-TRAILR2 humanized antibody-toxin-conjugate. The ADC has the specificity of TRAILR2 positive tumors, and after conjugated to TRAILR2, the positive tumors can be endocytosed into lysosomes of tumor cells, and is degraded by means of proteases inside the lysosomes to release free micromolecule urotoxins, so that different TRAILR2 positive tumor cells are killed specifically, the tumor growth is suppressed, the tumor cells are even completely eliminated, and the tumor is cured.

## Description

### Technical field

The present invention relates to the technical fields of biochemistry, immunochemistry, organic chemistry and medicinal chemistry. Specifically, the present invention relates to an antibody-toxin-conjugate (ADC, named Zapadcine-1, Zapadcine-3) and the pharmaceutical use thereof for treating tumor.

### Background

According to the "Global Cancer Report 2014" published by the WHO, the number of global cancer cases has grown rapidly, from 14 million in 2012 to 19 million in 2025 and 24 million in 2035. Both numbers of global cancer patients and deaths are disturbingly increasing. Nearly half of new cancer cases have occurred in Asia, most of which are in China. China has the highest number of new cancer cases. The three most common cancers in the world in 2012 were lung cancer (1.8 million), breast cancer (1.7 million), and colorectal cancer (1.4 million). The top three cancers in terms of lethality were lung cancer, liver cancer, and gastric cancer. Leukemia is one of the ten high-risk malignant tumors in China, and the mortality rate ranks sixth among all types of tumors. From 2008 to 2015, the market size of Chinese anti-tumor drugs increased steadily, and the market size increased from 28.986 billion yuan to 97.001 billion yuan. It is estimated that by 2018, the sales of this type of pharmaceutical market will reach 144.742 billion yuan.

Since the first therapeutic antibody (OKT3) was launched in 1986, many therapeutic antibodies and their derivatives have been used in clinical treatment, and more than 1,000 antibodies and their derivatives are in the research and development stage. Global therapeutic antibodies have grown from a very small market share at first to safe, specific and effective major disease treatment drugs that can be used to treat a variety of cancers, autoimmune diseases, transplant rejection, cardiovascular diseases, and various infectious diseases. In 2016, among the 16 new drugs approved by US FDA, 6 were antibody drugs. There are currently several drugs at phase II or III of clinical trials, and it is estimated that the US FDA will also approve 8-10 antibody drugs to be marketed in 2017. The average of annual sales of each antibody drug on the market is about 1 billion US dollars. Currently the so-called "bullet bombs" with annual sales of more than 1 billion US dollars are all antibody drugs. Therefore, therapeutic antibodies are the main direction of research and development of biotechnology drugs worldwide. Trapeutic antibodies are developing, not yet complete. Many scholars believe that therapeutic antibody drugs are "future medicine". However, in our country it is still lagging behind in the development and industrialization of therapeutic antibodies. So far, except for several imitative therapeutic antibody drugs, there are few innovative therapeutic antibody drugs with independent intellectual property rights.

Tumor necrosis factor superfamily (TNFSF) can induce apoptosis, in which three ligands such as FasL/Fas (CD95L/CD95), TNF/TNFR, and TRAIL/TRAILR and their receptors play an important role in inducing apoptosis of tumor cells. TRAIL is a type 2 transmembrane protein. Unlike the former two, TRAIL can specifically induce tumor cell apoptosis after binding to its corresponding death receptor without damaging normal cells. This characteristic of TRAIL and its death receptor has attracted great attention from researchers, and they are hoping to find a new method of tumor treatment based on it. There are five kinds of TRAIL receptors: TRAILR1, TRAILR2, TRAILR3, TRAILR4 and OPG. TRAILR1 and TRAILR2 are death receptors (DR). The intracellular region of death receptors has a complete death domain (DD), which can induce apoptosis of target cells. TRAILR3 and TRAILR4 are decoy receptors (DcR1, DcR2), which lack a complete death domain in the intracellular region and cannot transmit apoptosis signals. This is one of the mechanisms to protect normal cells from apoptosis. After the binding of TRAIL to its death receptor, it can stimulate a series of cascade reactions of aspartic proteases in the cell, and finally kills the tumor cells that are positive for TRAILR1 or TRAILR2.

Treatments of tumors targeting TRAILR1 or TRAILR2 comprises the agonistic monoclonal therapy using recombinant soluble TRAIL, anti-TRAILR1 or anti-TRAILR2, which have entered the human clinical trial stages (stage I/II) for the treatment of patients with various tumors. The results of clinical trials show that the safety is good, but the efficacy of the drug alone is not satisfactory. The reason may be related to the short half-life of recombinant soluble TRAIL *in vivo,* the low affinity of the therapeutic antibody used, the complex signaling pathways mediated by TRAILR1 or TRAILR2, and the failure to use effective molecular markers to select patients. Therefore, the current researches and developments of tumor therapeutic drugs targeting TRAILR1 or TRAILR2 aim to improve the stability and biological activity of TRAILR1 or TRAILR2 agonists, including combined use with chemical drugs, targeted drugs, antibody drugs and small molecule inhibitors, and the application of nanocarriers and other technologies, and some progress has been made.

Therefore, some international drug research and development companies and laboratories are adopting a drug combination regimen to conduct clinical trials of various tumor treatments, and have achieved good results. However, the cost of drug combination is high, especially when used in combination with chemical drugs, wherein the problem of high toxicity of chemical drugs has not been fundamentally solved. The patients' compliance is poor, and the toxic side effects on patients are still great.

### Summary of the invention

In view of the above shortcomings, the present invention creatively adopts the strategy of preparing an antibody-toxin-conjugate (ADC), which not only retains the high tumor specificity of the antibody, but also forms a conjugate complex using the linker to couple the antibody with the small molecule toxin. When the ADC specifically binds to a specific antigen on the surface of the tumor cell, it can bring the small molecule toxin into the lysosome in the tumor cell, and then release the toxin by proteolysis in the lysosome. Therefore, it specifically kills tumor cells with a greatly improved efficiency, and at the same time reduces the toxic and side effects of small molecular toxins, greatly improving safety, which makes it very popular.

In a first aspect of the present invention, it provides an antibody-drug conjugate or the pharmaceutically acceptable salt or solvent compound thereof, wherein the antibody-drug conjugate comprises:
(a) an antibody moiety; and
(b) a coupling moiety coupled to the antibody moiety, which is selected from the group consisting of a detectable label, a drug, a toxin, a cytokine, a radionuclide, an enzyme, and a combination thereof;
   wherein, the heavy chain variable region of the antibody comprises the following three complementary determining regions CDRs:
   (H1) CDRH1, shown in SEQ ID NO: 1,
   (H2) CDRH2, shown in SEQ ID NO: 2, and
   (H3) CDRH3, shown in SEQ ID NO: 3;
   wherein, any one of the amino acid sequences in the above heavy chain variable region amino acid sequence also includes a derivative sequence optionally added, deleted, modified, and/or substituted with at least one amino acid, and capable of retaining the binding affinity of TRAILR2; and/or
   the light chain variable region of the antibody comprises the following three complementary determining regions CDRs:
   (L1) CDRL1, shown in SEQ ID NO: 4,
   (L2) CDRL2, shown in SEQ ID NO: 5, and
   (L3) CDRL3, shown in SEQ ID NO: 6;
   wherein, any one of the amino acid sequences in the above light chain variable region amino acid sequence also includes a derivative sequence optionally added, deleted, modified, and/or substituted with at least one amino acid, and capable of retaining the binding affinity of TRAILR2.

In another preferred embodiment, the antibody comprises a complete antibody or an active fragment thereof.

In another preferred embodiment, the active fragment retains the binding activity to TRAILR2.

In another preferred embodiment, the antibody-drug conjugate ADC is represented by the following formula: 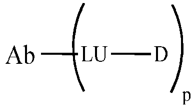 wherein,
Ab is an anti-TRAILR2 antibody;
LU is none or a linker connecting the antibody and the drug;
D is a drug;
p is the number of the drugs conjugated to the antibody; p is a value selected from 1-10, preferably 1-8, more preferably 2-4;
"-" is a bond or a linker.

In another preferred embodiment, the drug D is a toxin.

In another preferred embodiment, the toxin is a small molecule toxin.

In another preferred embodiment, the LU is a chemical linker.

In another preferred embodiment, the linker is a linker cleavable by a cathepsin.

In another preferred embodiment, the linker is a linker not cleavable by cathepsins.

In another preferred embodiment, the structure of the LU is shown in the following formula (I):

-L₁-L₂-L₃- (I)

wherein,
L₁ is none or Py, Mc;
L₂ is none or Vc, MAA, Mc;
L₃ is none or PAB, MAA, Mc;
each "-" is independently a key;
Py is 1,1',1"-(1,3,5-triazinane-1,3,5-triyl)tris(prop-2-en-1-one);
Vc is (S)-2-((S)-2-amino-3-methylbutanamido)-5-ureidopentanamide;
Mc is 6-(2,5-dioxocyclopent-3-en-1-yl) hexanoic acid;
PAB-OH is (4-aminophenyl) methanol;
MAA is 2-mercaptoacetic acid,
wherein the molecular structure is as follows: 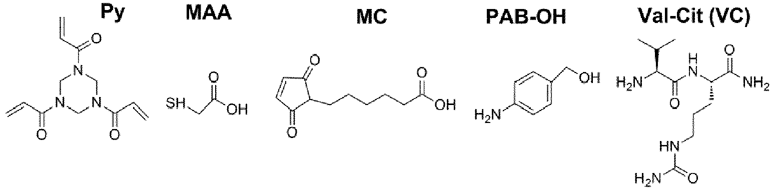 and at least one of L₁, L₂, L₃ is other than absent.

In another preferred embodiment, at least two of L1, L2, L3 are other than absent.

In another preferred embodiment, all of L1, L2, L3 are other than absent.

In another preferred embodiment, the LU is selected from the group consisting of: Py-Vc-PAB (Py-Vc-PAB-OH), Mc-Vc-PAB (Mc-Vc-PAB-OH), Py-MAA (Py-MAA-OH), and Mc (Mc-OH), wherein the molecular structure is as follows:

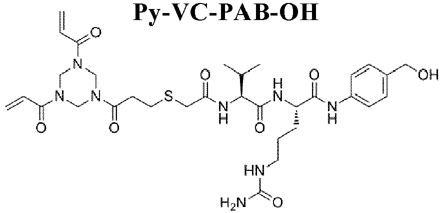

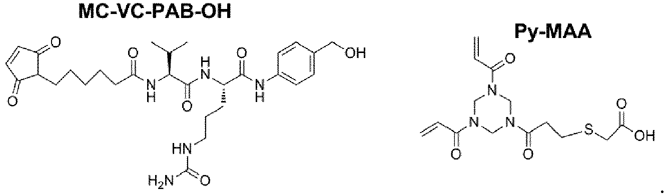

In another preferred embodiment, the L₁ in the linker LU is Py.

In another preferred embodiment, the LU is selected from the group consisting of: Py-Vc-PAB (Py-Vc-PAB-OH), Mc (Mc-OH) and Py-MAA (Py-MAA-OH).

In another preferred embodiment, the drug D (such as MMAE or MMAF or MMAD) is connected to the antibody (such as Zaptuzumab) via the linker LU, wherein the connection mode is selected from the group consisting of: bridge coupling and conventional coupling.

In another preferred embodiment, the bridge coupling is thiol bridge coupling (e.g., disulfide bridge coupling).

In another preferred embodiment, the conventional coupling is thiol conventional coupling.

In another preferred embodiment, the linker LU is connected to the drug D in a manner that can be cleaved by a protease.

In another preferred embodiment, the linker LU is connected to the drug D in a manner that cannot be cleaved.

In another preferred embodiment, the D is selected from the group consisting of: a chemotherapeutic agent, a radioactive substance, a toxin, an anti-cancer prodrug activating enzyme capable of converting the anti-cancer prodrugs into the active forms thereof, and a combination thereof.

In another preferred embodiment, the drug D is selected from the group consisting of: monomethyl auristatin F (MMAF), monomethyl auristatin E (MMAE), monomethyl auristatin D (MMAD) and the derivatives thereof, and a combination thereof

| | | |
|---|---|---|
| D1 | monomethyl auristatin F (MMAF) | 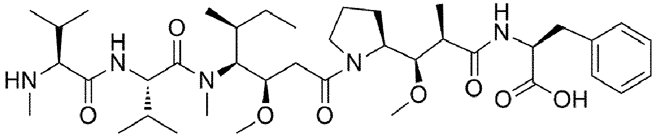 |
| D2 | monomethyl auristatin E (MMAE) | 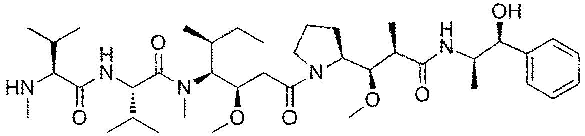 |
| D3 | monomethyl auristatin D (MMAD) | 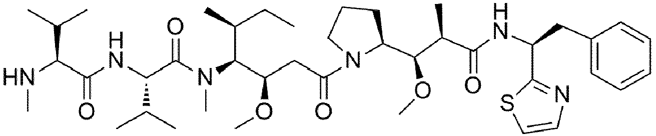 |

In another preferred embodiment, the amino acid residue linked to D is originally present in the antibody (parent antibody) or introduced exogenously.

In another preferred embodiment, the amino acid residue linked to D is a cysteine amino acid.

In another preferred embodiment, the cysteine amino acid is one or more free cysteine amino acids introduced in the parent antibody at one or more positions of the light chain according to the Kabat numbering rule and/or at one or more positions of the heavy chain according to the Kabat numbering rule, and at one or more positions of the heavy chain according to the EU numbering rules.

In another preferred embodiment, the amino acid residue linked to D is a lysine.

In another preferred embodiment, the active fragment is selected from the group consisting of: Fab, F(ab')2, Fv and scFv fragment.

In another preferred embodiment, the antibody is a monoclonal antibody (mAb).

In another preferred embodiment, the monoclonal antibody (mAb) is Zaptuzumab.

In another preferred embodiment, the antibody is an anti-TRAILR2 humanized monoclonal antibody.

In another preferred embodiment, the antibody includes: double-chain antibodies and single-chain antibodies.

In another preferred embodiment, the antibody is recombinant.

In another preferred embodiment, the antibody is produced in bacteria (such as *E. coli).*

In another preferred embodiment, the antibody is produced in eukaryotic cells (such as CHO cells).

In another preferred embodiment, the antibody is selected from: animal-derived antibodies, chimeric antibodies, humanized antibodies, fully humanized antibodies, and a combination thereof.

In another preferred embodiment, the antibody is a humanized antibody or a fully humanized antibody.

In another preferred embodiment, the antibody is an anti-tumor specific antibody.

In another preferred embodiment, the antibody is an antibody targeting the receptor TRAILR1 or TRAILR2 which is specifically expressed on tumor cells.

In another preferred embodiment, the antibody is selected from the group consisting of: anti-human tumor necrosis factor-related apoptosis inducing ligand (TRAIL) receptor 1 (TRAILR1) antibody, anti-human tumor necrosis factor-related apoptosis inducing ligand (TRAIL) receptor 2 (TRAILR2) antibody, and a combination thereof.

In another preferred embodiment, the antibody is a humanized monoclonal antibody against human tumor necrosis factor-related apoptosis inducing ligand (TRAIL) receptor 2 (TRAILR2).

In another preferred embodiment, the humanized monoclonal antibody against human tumor necrosis factor-related apoptosis inducing ligand (TRAIL) receptor 2 (TRAILR2) is Zaptuzumab.

In another preferred embodiment, the affinity of the antibody to human TRAILR2 protein EC₅₀ is 0.1-10nM, preferably 0.1-1.0nM, more preferably 0.1-0.5nM.

In another preferred embodiment, the antibody does not bind to the TRAILR2 protein from wild-type murine.

In another preferred embodiment, the antibody has one or more characteristics selected from the group consisting of:
(a) binding to TRAILR2 specific antigenic determinant;
(b) forming an antigen-antibody complex by binding to TRAILR2 on the cell surface, which can be endocytosed into the lysosome;
(c) inhibiting tumor formation and growth;
(d) inhibiting tumor cell migration or metastasis.

In another preferred embodiment, the antibody-drug conjugate (ADC) is selected from the group consisting of: Zapadcine-1a, Zapadcine-1b, Zapadcine-1c, Zapadcine-1d, Zapadcine-3a, Zapadcine-3b, Zapadcine-3c, Zapadcine-3d, and Zapadcine-3e; wherein,
the structure of conjugate Zapadcine-1a is as follows: 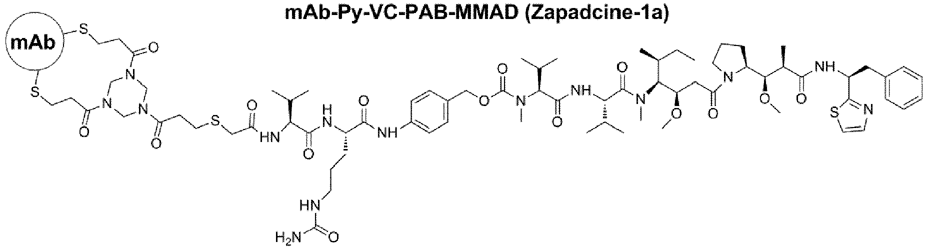 the structure of conjugate Zapadcine-1b is as follows: 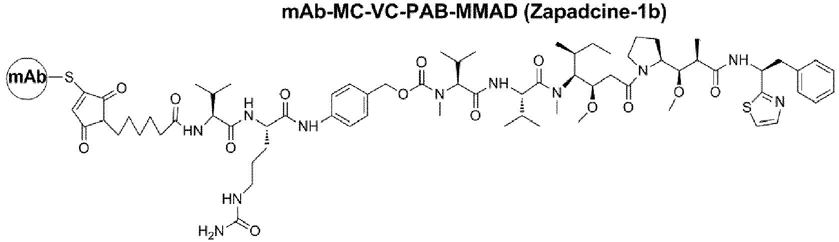 the structure of conjugate Zapadcine-1c is as follows: 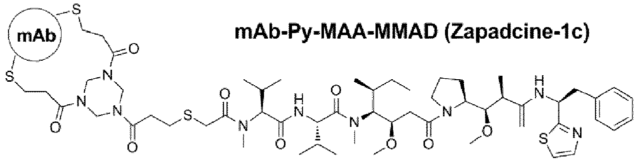 the structure of conjugate Zapadcine-1d is as follows: 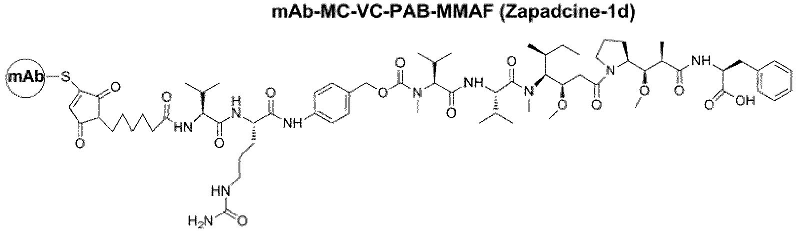 the structure of conjugate Zapadcine-3a is as follows: 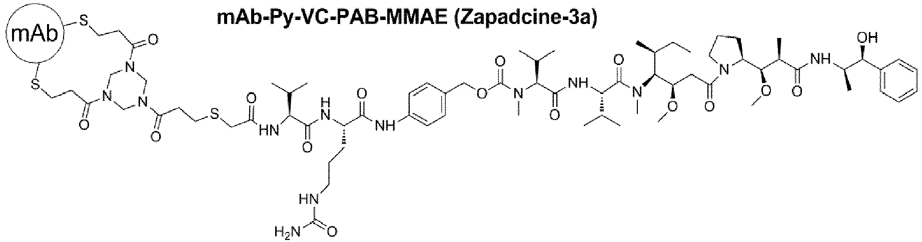 the structure of conjugate Zapadcine-3b is as follows: 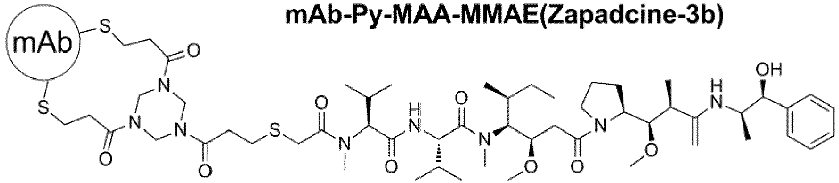 the structure of conjugate Zapadcine-3c is as follows: 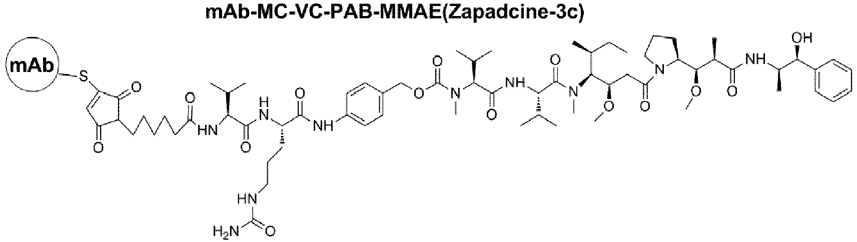 the structure of conjugate Zapadcine-3d is as follows: 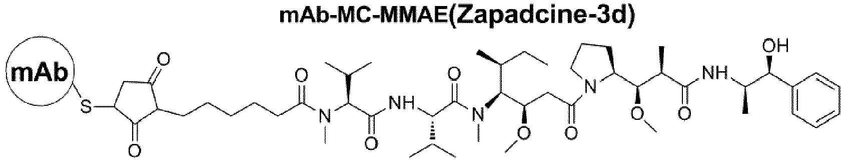 the structure of conjugate Zapadcine-3e is as follows:

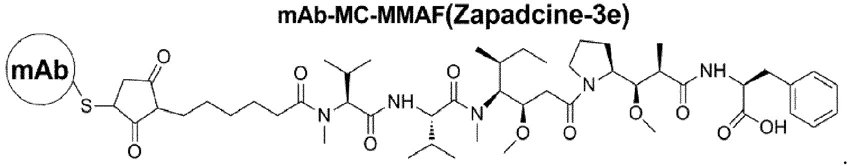

In another preferred embodiment, the antibody-drug conjugate (ADC) is selected from the following group: Zapadcine-1a and Zapadcine-1c; wherein,
the structure of conjugate Zapadcine-1a is as follows: 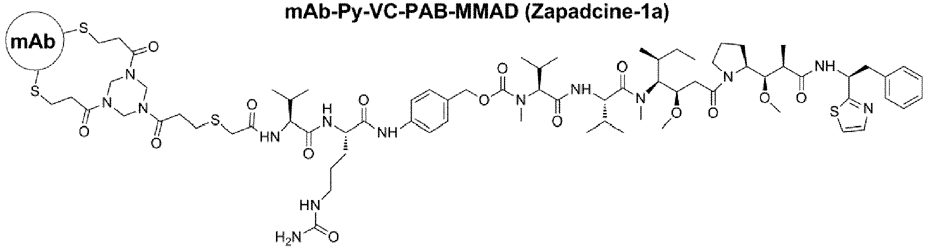 the structure of conjugate Zapadcine-1c is as follows:

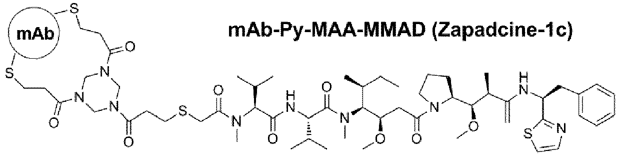

In another preferred embodiment, the antibody-drug conjugate (ADC) is selected from the group consisting of: Zapadcine-3a and Zapadcine-3d; wherein,
the structure of conjugate Zapadcine-3a is as follows: 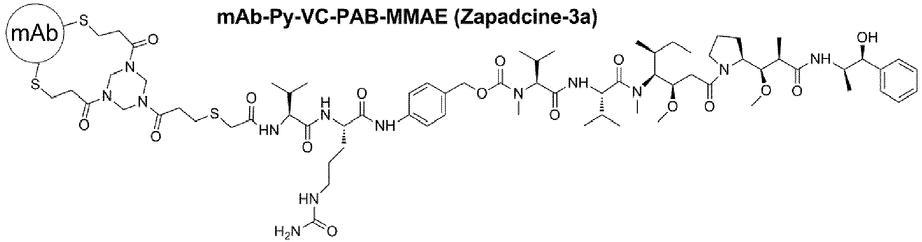 the structure of conjugate Zapadcine-3d is as follows:

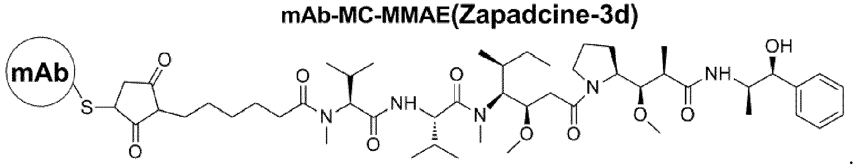

In another preferred embodiment, the amino acid sequence of the heavy chain variable region of the antibody is shown in SEQ ID No: 7; and/or, the amino acid sequence of the light chain variable region of the antibody is shown in SEQ ID No: 8;
wherein, the amino acid sequence of the heavy chain variable region further includes a derivative sequence with at least one amino acid optionally added, deleted, modified, and/or substituted, having a homology or sequence identity of at least 80% with the amino acid sequence shown in SEQ ID No: 7;
wherein, the amino acid sequence of the light chain variable region further includes a derivative sequence with at least one amino acid optionally added, deleted, modified, and/or substituted, having a homology or sequence identity of at least 80% with the amino acid sequence shown in SEQ ID No: 8.

In another preferred embodiment, the amino acid sequence of the heavy chain variable region of the monoclonal antibody (mAb) is shown in SEQ ID No: 7; and/or, the amino acid sequence of the light chain variable region of the antibody is shown in SEQ ID No: 8;
wherein, the amino acid sequence of the heavy chain variable region further includes a derivative sequence with at least one amino acid optionally added, deleted, modified, and/or substituted, having a homology or sequence identity of at least 80% with the amino acid sequence shown in SEQ ID No: 7;
wherein, the amino acid sequence of the light chain variable region further includes a derivative sequence with at least one amino acid optionally added, deleted, modified, and/or substituted, having a homology or sequence identity of at least 80% with the amino acid sequence shown in SEQ ID No: 8;
the antibody-drug conjugate (ADC) is selected from the following group: Zapadcine-3a and Zapadcine-3d.

In another preferred embodiment, the antibody-drug conjugate (ADC) is selected from the following group: Zapadcine-3a and Zapadcine-3d; wherein the monoclonal antibody (mAb) is Zaptuzumab.

In another preferred embodiment, the antibody-drug conjugate (ADC) is Zapadcine-3a, wherein the monoclonal antibody (mAb) is Zaptuzumab.

In another preferred embodiment, the amino acid sequence of the heavy chain variable region of the monoclonal antibody (mAb) is shown in SEQ ID No: 7; and/or, the amino acid sequence of the light chain variable region of the antibody is shown in SEQ ID No: 8;
wherein, the amino acid sequence of the heavy chain variable region further includes a derivative sequence with at least one amino acid optionally added, deleted, modified, and/or substituted, having a homology or sequence identity of at least 80% with the amino acid sequence shown in SEQ ID No: 7;
wherein, the amino acid sequence of the light chain variable region further includes a derivative sequence with at least one amino acid optionally added, deleted, modified, and/or substituted, having a homology or sequence identity of at least 80% with the amino acid sequence shown in SEQ ID No: 8;
the antibody-drug conjugate (ADC) is selected from the following group: Zapadcine-1a and Zapadcine-1c.

In another preferred embodiment, the antibody-drug conjugate (ADC) is selected from the following group: Zapadcine-1a and Zapadcine-1c; wherein the monoclonal antibody (mAb) is Zaptuzumab.

In another preferred embodiment, the antibody-drug conjugate (ADC) is Zapadcine-1a, wherein the monoclonal antibody (mAb) is Zaptuzumab.

In a second aspect of the present invention, it provides use of the antibody-drug conjugate or the pharmaceutically acceptable salt or solvent compound thereof according to the first aspect of the present invention, for (i) preparing diagnostic reagents; and/or (ii) preparing drugs for preventing and/or treating a TRAILR2 related disease.

In another preferred embodiment, the TRAILR2 related disease is selected from the group consisting of: the occurrence, growth, and/or metastasis of tumors (e.g., TRAILR2 positively expressing tomors).

In another preferred embodiment, theTRAILR2 positively expressing tomor is a TRAILR2 positively expressing cancer.

In another preferred embodiment, the cancer is selected from: T lymphocytic leukemia, B lymphocytic leukemia, non-T and non-B lymphocytic leukemia, non-small cell lung cancer, liver cancer, colon cancer, breast cancer, ovarian cancer, pancreatic cancer, thyroid cancer, head and neck squamous cell carcinoma, esophageal squamous cell carcinoma, lung squamous cell carcinoma, lung adenocarcinoma, cervical squamous cell carcinoma, pancreatic squamous cell carcinoma, colon squamous cell carcinoma, gastric squamous cell carcinoma, prostate cancer, osteosarcoma and soft tissue sarcoma.

In another preferred embodiment, the cancer is a TRAILR2 positively expressing cancer and the cancer is selected from: T lymphocytic leukemia, B lymphocytic leukemia, non-T and non-B lymphocytic leukemia, non-small cell lung cancer, liver cancer, colon cancer, breast cancer, ovarian cancer, pancreatic cancer, thyroid cancer, head and neck squamous cell carcinoma, esophageal squamous cell carcinoma, lung squamous cell carcinoma, lung adenocarcinoma, cervical squamous cell carcinoma, pancreatic squamous cell carcinoma, colon squamous cell carcinoma, gastric squamous cell carcinoma, prostate cancer, osteosarcoma and soft tissue sarcoma.

In another preferred embodiment, the TRAILR2 positive expression means that the ratio of the TRAILR2 transcript and/or protein level L1 in a tumor tissue and/or a tumor cell to the TRAILR2 transcript and/or protein level L0 in a normal tissue and/or a normal cell, L1/L0, is ≥2, preferably ≥3.

In a third aspect of the present invention, it provides a pharmaceutical composition, comprising:
(i) an active ingredient, which is the antibody-drug conjugate or the pharmaceutically acceptable salt or solvent compound thereof according to the first aspect of the invention, or a combination thereof; and
(ii) a pharmaceutically acceptable carrier.

In another preferred embodiment, the active ingredient is Zapadcine-3a, Zapadcine-3d or a combination thereof, wherein the monoclonal antibody (mAb) is Zaptuzumab.

In another preferred embodiment, the active ingredient is Zapadcine-3a, wherein the monoclonal antibody (mAb) is Zaptuzumab.

In another preferred embodiment, the active ingredient is Zapadcine-1a, Zapadcine-1c or a combination thereof, wherein the monoclonal antibody (mAb) is Zaptuzumab.

In another preferred embodiment, the active ingredient is Zapadcine-1a, wherein the monoclonal antibody (mAb) is Zaptuzumab.

In another preferred embodiment, the pharmaceutical composition is in a unit dosage form for human use.

In another preferred embodiment, the pharmaceutical composition is a liquid preparation.

In another preferred embodiment, in the pharmaceutical composition, the content of the antibody-drug conjugate is 0.005-50wt%, preferably 0.05-10wt% .

In another preferred embodiment, the pharmaceutical composition also comprises (iii) additional therapeutic agents.

In another preferred embodiment, the additional therapeutic agent includes a chemotherapeutic agent.

In a fourth aspect of the present invention, it provides an *in vitro* non-therapeutic method for inhibiting tumor cells, comprising the steps of: contacting the tumor cells with the antibody-drug conjugate or the pharmaceutically acceptable salt or solvent compound thereof according to the first aspect of the present invention.

In another preferred embodiment, the contacting is performed in an *in vitro* culture system.

In a fifth aspect of the present invention, it provides a method for preventing and/or treating tumors, comprising the steps of: administering to the subject in need the antibody-drug conjugate or the pharmaceutically acceptable salt or solvent compound thereof according to the first aspect of the invention or the pharmaceutical composition according to the third aspect of the present invention.

In another preferred embodiment, the subject is a mammal, including a human.

In a sixth aspect of the present invention, it provides a method for alleviating the growth of tumors in a subject, comprising the steps of: combining an effective amount of the antibody-drug conjugate or the pharmaceutically acceptable salt or solvent compound thereof according to the first aspect of the invention with one or more treatments selected from the group consisting of: radiation treatment, chemotherapeutic treatment, biological treatment, and a combination thereof.

In a seventh aspect of the present invention, it provides use of the antibody-drug conjugate or the pharmaceutically acceptable salt or solvent compound thereof according to the first aspect of the invention or the pharmaceutical composition according to the third aspect of the present invention in the preparation of a medicament for preventing and/or treating tumors.

It should be understood that within the scope of the present invention, the various technical features of the present invention above and the various technical features specifically described hereinafter (as in the embodiments) may be combined with each other to constitute a new or preferred technical solution. Due to space limitations, it is not repeated here.

### Brief description of the figures

Figure 1 is the molecular structural formula of the chemical linker of the present invention.
Figure 2 is the molecular structure formula of the anti-TRAILR2 antibody-drug conjugate of the present invention.
Figure 3 is the molecular structural formula of the chemical toxin of the present invention.
Figure 4 shows the affinity of Zapadcine-1a of the present invention to the recombinant proteinTRAILR2 (antigen).
Figure 5 shows the inhibitory effect of Zapadcine-1a of the present invention on the growth of lymphoblastic leukemia Jurkat E6-1 subcutaneous xenografts in nude mice.
Figure 6 shows the inhibitory effect of Zapadcine-1a of the present invention on the growth of large cell lung cancer NCI-H460 xenografts in nude mice.
Figure 7 shows the inhibitory effect of Zapadcine-1a of the present invention on the growth of human non-small cell lung cancer NCI-H1975 xenografts in nude mice.
Figure 8 shows the inhibitory effect of Zapadcine-1a of the present invention on the growth of hepatoma cell SMMC-7721 xenografts in nude mice.
Figure 9 shows the inhibitory effect of Zapadcine-1a of the present invention on the growth of ovarian cancer cell A2780 xenografts in nude mice.
Figure 10 shows the inhibitory effect of Zapadcine-1a of the present invention on the growth of pancreatic cancer cell Mia PaCa-2 xenografts in nude mice.
Figure 11 shows the maximum tolerated dose of Zapadcine-1a of the present invention in a single administration to normal mice.
Figure 12 shows the affinity of Zapadcine-3a of the present invention to the recombinant protein TRAILR2 (antigen).
Figure 13 shows the inhibitory effect of Zapadcine-3a of the present invention on the growth of human non-small cell lung cancer MSTO-211H xenografts in nude mice.
Figure 14 shows the inhibitory effect of Zapadcine-3a of the present invention on the growth of pancreatic cancer cell Mia PaCa-2 xenografts in nude mice.
Figure 15 shows the inhibitory effect of Zapadcine-3a of the present invention on the growth of T lymphocyte leukemia cell Jurkat E6-1 xenografts in nude mice.
Figure 16 shows the inhibitory effect of Zapadcine-3a of the present invention on the growth of non-T and non-B lymphocytic leukemia cell Reh xenografts in nude mice.
Figure 17 shows the maximum tolerated dose of Zapadcine-3a of the present invention in a single administration to normal rats.
Figure 18 shows the maximum tolerated dose of Zapadcine-3a of the present invention in a single administration of normal cynomolgus monkeys.

### Detailed description

By extensively and intensively studies, the present inventors have designed antibody-drug conjugates targeting TRAILR2, and the antibody-drug conjugates have significant anti-tumor effects. The present invention also provides the pharmaceutical use of the anti-TRAILR2 antibody-drug conjugate and its use for inhibiting or preventing tumors.

The anti-TRAILR2 (TRAILR2 or CD262) humanized monoclonal antibody of the present invention has a unique gene sequence, antigenic determinant and a very strong antigen affinity compared with other antibodies against the target of TRAILR2 that have entered human clinical trials at home and abroad, and can specifically kill a variety of TRAILR2-positive tumor cells *in vivo* and *in vitro,* and inhibit the growth of tumor cells, but it is almost non-toxic to normal cells and tissues with good safety.

In a preferred embodiment of the present invention, and anti-TRAILR2 humanized monoclonal antibody (Zaptuzumab) was used, and disulfide bridge coupling technology was used to couple Zaptuzumab to small molecule toxins through different chemical linkers. A variety of ADCs with different chemical structures were obtained. After repeated screening of anti-tumor activity *in vivo* and *in vitro,* ADC drug candidates with excellent druggability (named Zapadcine-1 and Zapadcine-3) were obtained, which can be used for treatment of multiple TRAILR2-positive tumors.

### The terms

Explanation of the core terms of the present invention

| | | |
|---|---|---|
| ADC | antibody-drug conjugate | antibody-toxin conjugate |
| | linker | linker |
| MC | Maleimidocaproyl | Maleimidocaproyl |
| VC | Valine-Citrulline | Valine-Citrulline |
| MMAD | monomethylauristatin D | monomethylauristatin D |
| MMAE | monomethylauristatin E | monomethylauristatin E |
| MMAF | monomethylauristatin F | monomethylauristatin F |
| DM1 | Maytansinoid DM1 | Maytansinoid DM1 |
| DM4 | Maytansinoid DM4 | Maytansinoid DM4 |
| | calicheamicin | calicheamicin |
| | duocarmycin | duocarmycin |
| | Duomycin7 | Duomycin7 |
| TNFSF | tumor necrosis factor superfamily | tumor necrosis factor superfamily |
| TNF | tumor necrosis factor | tumor necrosis factor |
| TNFR | tumor necrosis factor receptor | tumor necrosis factor receptor |
| TRAIL | tumor necrosis factor-related apoptosis-inducing ligand | tumor necrosis factor-related apoptosis-inducing ligand |
| TRAILR | tumor necrosis factor-related apoptosis-inducing ligand receptor | tumor necrosis factor-related apoptosis-inducing ligand receptor |
| DR | death receptor | death receptor |
| DD | death domain | death domain |
| DcR | decoy receptor | decoy receptor |
| | caspases | caspases |
| | epitope | antigenic determinant |
| SPF | specific pathogen free | specific pathogen free |
| PBMC | peripheral blood mononuclear cell | peripheral blood mononuclear cell |
| | surviving rate | surviving rate |
| PBS | phosphate buffer saline | phosphate buffer saline |
| NOD-SCID | no obese diabetic/severe combined immunodeficiency | no obese diabetic/severe combined immunodeficiency |
| | vehicle | negative control |
| | vincristin | vincristin |
| | paclitaxel | paclitaxel |
| | epirubicin-HCl | Epirubicin-HCl |
| Q3D | every three days | every three days |
| QW (Q7D) | once a week | once a week |
| i.v. | intravenous(ly) | intravenous(ly) |
| ANOVA | analysis of variance | analysis of variance |
| RT | room temperature | room temperature |
| BW | body weight | body weight |
| TV | tumor volume | tumor volume |
| TW | tumor weight | tumor weight |
| TGI | tumor growth inhibition | tumor growth inhibition |
| FFPE | formalin fixed paraffin embedded | formalin fixed paraffin embedded |
| N/A | not available or not applicable | not available or not applicable |
| SD | standard deviation | standard deviation |
| SEM | standard error of mean | standard error of mean |
| IC50 | half maximal inhibitory concentration | half maximal inhibitory concentration of the drug tested |
| Eff. | Efficacy | Efficacy |
| TMB | 3,3',5,5'- Tetramethylbenzidine | 3,3',5,5'-tetramethylbenzidine |
| | Zaptuzumab | Zaptuzumab |

As used herein, the terms "antibody-drug conjugate", "antibody conjugate", "antibody drug conjugate", "antibody-drug conjugates" and "immunoconjugate" can be used interchangeably and refer to the conjugate formed by (a) the antibody or its active fragment and (b) the drug.

As used herein, the terms "antibody-drug conjugate of the present invention", "the conjugate of antibody and drug of the present invention" and "ADC of the present invention" can be used interchangeably and refer to the conjugates formed by the antibodies or active fragments against TRAILR2 of the present invention and drugs.

Unless otherwise defined, all technical and scientific terms used herein have the same meaning as commonly understood by those ordinarily skilled in the art to which this invention belongs. As used herein, when referring to specifically recited values, the term "about" means that the value can vary from the recited value by no more than 1%. For example, the expression "about 100" includes all values between 99 and 101 (e.g., 99.1, 99.2, 99.3, 99.4, etc.).

### Antibody

As used herein, the term "antibody" or "immunoglobulin" is a heterotetrameric glycoprotein of about 150,000 daltons with the same structural characteristics, which consists of two identical light chains (L) and two identical heavy chains (H). Each light chain is connected to the heavy chain through a covalent disulfide bond, and the numbers of disulfide bonds between heavy chains of different immunoglobulin isotypes are different. Each heavy and light chain also has regularly spaced intrachain disulfide bonds. Each heavy chain has a variable region (VH) at one end, followed by multiple constant regions. Each light chain has a variable region (VL) at one end and a constant region at the other end; the constant region of the light chain is opposite to the first constant region of the heavy chain, and the variable region of the light chain is opposite to the variable region of the heavy chain. Special amino acid residues form an interface between the variable regions of the light and heavy chains.

As used herein, the term "variable" means that certain parts of the variable region of an antibody differ in sequence, which forms the binding and specificity of various specific antibodies for their specific antigens. However, the variability is not evenly distributed throughout the variable region of the antibody. It is concentrated in three segments called complementary determining regions (CDRs) or hypervariable regions in the light chain and heavy chain variable regions. The more conserved part of the variable region is called the framework region (FR). The variable regions of the natural heavy and light chains each contain four FR regions, which are roughly in the β-sheet configuration, connected by the three CDRs that form the connecting loop, and in some cases may form a partial β-sheet structure. The CDRs in each chain get close through the FR regions and form the antigen-binding site of the antibody together with the CDRs in another chain (see Kabat et al., NIH Publ. No. 91-3242, Volume I, pages 647-669 (1991)). The constant regions are not directly involved in the binding of antibodies to antigens, but they exhibit different effector functions, such as involve in the antibody-dependent cytotoxicity of antibodies.

The light chains of vertebrate antibodies (immunoglobulins) can be classified into one of two distinct classes (referred to as κ and λ) based on the amino acid sequence of their constant regions. Immunoglobulins can be divided into different types, according to the amino acid sequence of the constant region of the heavy chain. There are five main classes of immunoglobulins: IgA, IgD, IgE, IgG, and IgM, some of which can be further divided into subclasses (isotypes), such as IgG1, IgG2, IgG3, IgG4, IgA, and IgA2. The heavy chain constant regions corresponding to different classes of immunoglobulins are called α, δ, ε, γ, and µ. The subunit structures and three-dimensional configurations of different classes of immunoglobulins are well known to those skilled in the art.

In general, the antigen-binding properties of an antibody can be described by the three specific regions located in the variable regions of the heavy and light chains, called complementary determining regions (CDRs), which divide this segment into 4 framework regions (FRs). The amino acid sequences of the four FRs are relatively conservative and do not directly participate in the binding reaction. These CDRs form a circular structure, and get close to each other in space structure through the β-sheets formed by the FRs in between. The CDRs on the heavy chain and the CDRs on the corresponding light chain constitute the antigen binding site of the antibody. The amino acid sequences of antibodies of the same type can be compared to determine which amino acids constitute the FR or CDR regions.

The present invention includes not only intact antibodies, but also immunologically active fragments of antibodies (such as antigen-binding fragments) or fusion proteins formed by antibodies and other sequences. Therefore, the present invention also includes fragments, derivatives and analogs of the antibodies.

In the present invention, antibodies include murine, chimeric, humanized, or fully human antibodies prepared by techniques well known to those skilled in the art. Recombinant antibodies, such as chimeric and humanized monoclonal antibodies, including human and non-human parts, can be obtained by standard DNA recombination techniques, and they are all useful antibodies. A chimeric antibody is a molecule in which different parts are derived from different animal species, such as a chimeric antibody with a variable region from a monoclonal antibody from a mouse and a constant region from a human immunoglobulin (see, for example, US Patent No. 4,816,567 and US Patent 4,816,397, hereby incorporated by reference in its entirety). Humanized antibodies refer to antibody molecules derived from non-human species, having one or more complementary determining regions (CDRs) derived from non-human species and framework regions derived from human immunoglobulin molecules (see US Patent 5,585,089, hereby incorporated by reference in its entirety). These chimeric and humanized monoclonal antibodies can be prepared using recombinant DNA techniques well known in the art.

In the present invention, the antibody may be monospecific, bispecific, trispecific, or more multispecific.

In the present invention, the antibody of the present invention also includes conservative variants thereof, which means that compared with the amino acid sequence of the antibody of the present invention, there are at most 10, preferably at most 8, more preferably at most 5, most preferably at most 3 amino acids replaced by amino acids with the same or similar properties to form a polypeptide. These conservatively variant polypeptides are preferably produced by amino acid substitution according to Table 1.

**Table 1**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-TRAILR2 antibody of the present invention

The present invention provides an antibody with high specificity and high affinity against TRAILR2, which comprises a heavy chain and a light chain, wherein the heavy chain contains a heavy chain variable region (VH) amino acid sequence, and the light chain contains a light chain variable region (VL) amino acid sequence.

Preferably, the respective CDRs of the heavy chain variable region (VH) amino acid sequence and the light chain variable region (VL) amino acid sequence are selected from the group consisting of:
a1) SEQ ID NO: 1: CDRH1, DFSMN;
a2) SEQ ID NO: 2: CDRH2, WINTETGEPTYADDFKG;
a3) SEQ ID NO: 3: CDRH3, IDY;
a4) SEQ ID NO: 4: CDRL1, RSSQSLVHSNGNTYLH;
a5) SEQ ID NO: 5: CDRL2, KVSNRFS;
a6) SEQ ID NO: 6: CDRL3, FQSTHVPHT;
a7) a sequence having TRAILR2 binding affinity with at least one amino acid added, deleted, modified and/or substituted in any of the above amino acid sequences.

In another preferred embodiment, the sequence with at least one amino acid added, deleted, modified and/or substituted in any of the above amino acid sequences is preferably an amino acid sequence having a homology of at least 80%, more preferably at least 85%, more preferably at least 90%, most preferably at least 95% to the above amino acid sequence.

Preferably, the antibody has the activity of activating TRAILR2 related signaling pathway; the activity of promoting cell apoptosis; the activity of inhibiting cell proliferation; the activity of promoting cell autophagy, or a combination thereof.

Typically, the present invention provides an antibody against TRAILR2 comprising: the heavy chain variable region of the present invention; and/or the light chain variable region of the present invention;
wherein, the heavy chain variable region of the antibody comprises the following three complementary determining regions CDRs:
CDRH1 shown in SEQ ID NO: 1,
CDRH2 shown in SEQ ID NO: 2, and
CDRH3 shown in SEQ ID NO: 3;
wherein, any one of the above amino acid sequences also includes a derivative sequence that is optionally with at least one amino acid added, deleted, modified, and/or substituted, and is capable of retaining the binding affinity of TRAILR2;
the light chain variable region of the antibody comprises the following three complementary determining regions CDRs:
CDRL1 shown in SEQ ID NO: 4,
CDRL2 shown in SEQ ID NO: 5, and
CDRL3 shown in SEQ ID NO: 6;
a derivative sequence having TRAILR2 binding affinity with at least one amino acid added, deleted, modified and/or substituted in any of the above amino acid sequences.

Preferably, the sequence of the heavy chain variable region of the antibody is SEQ ID NO: 7; and/or the sequence of the light chain variable region of the antibody is SEQ ID NO: 8.

In the present invention, the antibody is selected from: an animal-derived antibody, a chimeric antibody, a humanized antibody, a fully human antibody, and a combination thereof.

In another preferred embodiment, the number of added, deleted, modified and/or substituted amino acids does not exceed 40% of the total number of amino acids in the original amino acid sequence.

In another preferred embodiment, the number of amino acids added, deleted, modified and/or substituted is 1-7.

In another preferred embodiment, the amino acid sequence with at least one amino acid added, deleted, modified and/or substituted is an amino acid sequence having a homology of at least 80% to the above amino acid sequence.

In another preferred embodiment, the amino acid sequence with at least one amino acid added, deleted, modified, and/or substituted has the activity to activate the TRAILR2 related signaling pathway; and have one or more of the abilities to promote cell apoptosis, to inhibit cell proliferation, and to promote cell autophagy.

The antibody of the present invention may be a double-chain or single-chain antibody, and may be selected from an animal-derived antibody, a chimeric antibody and a humanized antibody, more preferably be selected from a humanized antibody and a human-animal chimeric antibody (such as human-mouse chimeric antibodies), more preferably a fully human antibody.

The antibody derivatives of the present invention may be single chain antibodies, and/or antibody fragments, such as: Fab, Fab', (Fab')2 or other known antibody derivatives in the art, etc., as well as any one or several of IgA, IgD, IgE, IgG and IgM antibodies or other subtypes.

Wherein, the animal is preferably a mammal, such as a rat.

The antibody of the present invention may be a chimeric antibody, a humanized antibody, a CDR grafted and/or modified antibody targeting human TRAILR2.

In a preferred embodiment of the present invention, any one or several of the above SEQ ID No: 1 - SEQ ID No: 3, or a sequence with at least one amino acid added, deleted, modified and/or substituted thereof and with TRAILR2 binding affinity is located in the CDR region of the heavy chain variable region (VH).

In a preferred embodiment of the present invention, any one or several of the above SEQ ID No: 4 - SEQ ID No: 6, or a sequence with at least one amino acid added, deleted, modified and/or substituted thereof and with TRAILR2 binding affinity is located in the CDR region of the light chain variable region (VL).

In a more preferred embodiment of the present invention, VH CDR1, CDR2 and CDR3 are independently selected from any one or several sequences of SEQ ID No: 1 - SEQ ID No: 3, and sequences with at least one amino acid added, deleted, modified and/or substituted thereof and with TRAILR2 binding affinity; VL CDR1, CDR2 and CDR3 are independently selected from any one or several sequences of SEQ ID No: 4 - SEQ ID No: 6, and sequences with at least one amino acid added, deleted, modified and/or substituted thereof and with TRAILR2 binding affinity.

In the above content of the present invention, the number of added, deleted, modified and/or substituted amino acids is preferably not more than 40% of the total number of amino acids in the original amino acid sequence, more preferably not more than 35%, more preferably 1-33% , more preferably 5-30%, more preferably 10-25%, more preferably 15-20%.

In the above content of the present invention, more preferably, the number of added, deleted, modified and/or substituted amino acids may be 1-7, more preferably 1-5, more preferably 1-3, more preferably 1-2.

In another preferred embodiment, the antibody targeting TRAILR2 is Zaptuzumab.

In another preferred embodiment, the amino acid sequence of the heavy chain variable region (VH) of the antibody Zaptuzumab is the amino acid sequence shown in SEQ ID NO: 7.

In another preferred embodiment, the amino acid sequence of the light chain variable region (V-Kappa) of the antibody Zaptuzumab is the amino acid sequence shown in SEQ ID NO: 8.

In another preferred embodiment, the amino acid sequence of the heavy chain variable region of the antibody further includes a derived amino acid sequence with at least one amino acid optionally added, deleted, modified, and/or substituted, and with at least 80% homology or sequence identity with the amino acid sequence shown in SEQ ID No: 7.

In another preferred embodiment, the amino acid sequence of the light chain variable region of the antibody further includes a derived amino acid sequence with at least one amino acid optionally added, deleted, modified, and/or substituted, and with at least 80% homology or sequence identity with the amino acid sequence shown in SEQ ID No: 8.

In a specific embodiment, the homology or sequence identity may be 80% or more, preferably 90% or more, more preferably 95%-98%, and most preferably 99% or more.

Methods known to those of ordinary skill in the art for determining sequence homology or identity include, but are not limited to: Computational Molecular Biology, edited by Lesk, A.M., Oxford University Press, New York, 1988; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987, and Sequence Analysis Primer, edited by Gribskov, M. and Devereux, J., Stockton Press, New York, 1991, and Carillo, H. and Lipman, D., SIAM J. Applied Math., 48:1073 (1988). The preferred method of determining identity is to obtain the greatest match between the sequences tested. The method of determining identity is compiled in a publicly available computer program. Preferred computer program methods for determining the identity between two sequences include, but are not limited to: GCG package (Devereux, J. et al., 1984), BLASTP, BLASTN, and FASTA (Altschul, S, F. et al., 1990). The BLASTX program is available to the public from NCBI and other sources (BLAST Manual, Altschul, S. et al., NCBI NLM NIH Bethesda, Md. 20894; Altschul, S. et al., 1990). The well-known Smith Waterman algorithm can also be used to determine identity.

### Preparation of antibodies

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

It has been possible now to obtain a DNA sequence encoding the antibody (or a fragment thereof, or a derivative thereof) according to the present invention completely by chemical synthesis. Then, the DNA sequence can be introduced into various existing DNA molecules (or, for example, vectors) and cells known in the art. In addition, mutations can also be introduced into the protein sequence according to the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an in vitro binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem., 107: 220 (1980)).

The antibody according to the present invention can be expressed in a cell or on the cell membrane, or is secreted extracellularly. If necessary, the recombinant protein can be separated and purified by various separation methods according to its physical, chemical, and other properties. These methods are well known to those skilled in the art. Examples of these methods include, but are not limited to, conventional renaturation treatment, treatment with a protein precipitant (salting out method), centrifugation, osmotic bacteria disruption, ultrasonic treatment, ultracentrifugation, molecular sieve chromatography (gel filtration), adsorption chromatography, ion exchange chromatography, high performance liquid chromatography (HPLC), various other liquid chromatographic techniques, and combinations of these methods.

### Cytotoxic Agents

Drugs, which can be used to form the ADC according to the present invention, include, but are not limited to: cytotoxic agents.

The term "cytotoxic agents" refer to substances that inhibit or block cell expression activity, cell function and/or result in cell destruction. The term includes radioisotopes, chemotherapeutics, and toxins, such as small-molecular toxins or enzymatically active toxins (including fragments and/or variants thereof) derived from bacteria, fungi, plants or animals. Examples of cytotoxic agents include, but are not limited to: auristatins (e.g. auristatin E, auristatin F, MMAE and MMAF), chlortetracycline, maytansinols, ricin, ricin A-chain, cobstatin, duocarmycin, dolastatin, doxorubicin, daunorubicin, paclitaxel, cisplatin, cc 1065, ethidium bromide, mitomycin, etoposide, tenoposide, vincristine, vinblastine, colchicine, dihydroxy anthrax dione, actinomycin, diphtheria toxin, Pseudomonas exotoxin (PE) A, PE40, Acacia toxin, Acacia toxin A chain, modeccin A chain, α-sarcina, gelonin, mitogellin, retstrictocin, phenomycin, enomycin, curicin, crotin, calicheamicin, Sapaonaria officinalis inhibitor, glucocorticoid and other chemotherapeutic agents, as well as radioisotopes such as At211, 1131, 1125, Y90, Re186, Re188, Sm153, Bi212 or 213, P32 and the radioisotopes of Lu including Lu177. An antibody can also be conjugated to an anti-cancer prodrug-activating enzyme that can convert the prodrug into its active form.

A preferred small-molecule drug is a compound having high cytotoxicity, preferably monomethyl auristatin, galactomycin, maytansines, or a combination thereof; more preferably is selected from: monomethyl auristatin E (MMAE), monomethyl auristatin-D (MMAD), monomethyl auristatin-F (MMAF), or a combination thereof.

### Antibody-Drug Conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Typical conjugation manners suitable for the present invention include both K-Lock and C-Lock conjugation manners. In the K-Lock conjugation manner, a drug molecule is conjugated to the lysine (K) residue in an antibody sequence; in the C-Lock conjugation manner, a drug molecule is coupled to the cysteine (C) residue in an antibody sequence.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH3COO-, Cl- or NO3-; and polymethylene dimethyl sulfide thiosulfonate. The linker may include, for example, a maleimide linked to an antibody via thiosuccimide.

A drug may be any cytotoxic, cytostatic or immunosuppressive drug. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycindocamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows: 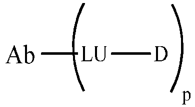 wherein,
Ab is an anti-TRAILR2 antibody;
LU is none or a linker connecting the antibody and the drug;
D is a drug;
p is the number of the drugs conjugated to the antibody; p is a value selected from 1-10, preferably 1-8, more preferably 2-4;
"-" is a bond or a linker.

Typically, the drug parts (such as toxins), linkers, connection modes, and cleavage modes of the four ADCs of the present invention are shown in Table 2:

**Table 2**

| name | Zapadcine-1a | Zapadcine-1b | Zapadcine-1c | Zapadcine-1d |
|---|---|---|---|---|
| toxin | MMAD | MMAD | MMAD | MMAF |
| linker | Py-Vc-PAB | Mc-Vc-PAB | Py-MAA | Mc-Vc-PAB |
| Connection mode | Thiol bridge coupling | Thiol conventional coupling | Thiol bridge coupling | Thiol conventional coupling |
| Cleavage mode | Cleavable | Cleavable | Non-cleavable | Cleavable |

Typically, the drug parts (such as toxins), linkers, connection modes, and cleavage modes of the five ADCs of the present invention are shown in Table 3:

**Table 3**

| name | Zapadcine-3 a | Zapadcine-3b | Zapadcine-3 c | Zapadcine-3d | Zapadcine-3 e |
|---|---|---|---|---|---|
| toxin | MMAE | MMAE | MMAE | MMAE | MMAF |
| linker | PY-VC-PAB | PY-MAA | MC-VC-PAB | MC | MC |
| Connection mode | Thiol bridge coupling | Thiol bridge coupling | Thiol Conventional coupling | Thiol Conventional coupling | Thiol Conventional coupling |
| Cleavage mode | Cleavable | Non-cleavable | Cleavable | Non-cleavable | Non-cleavable |

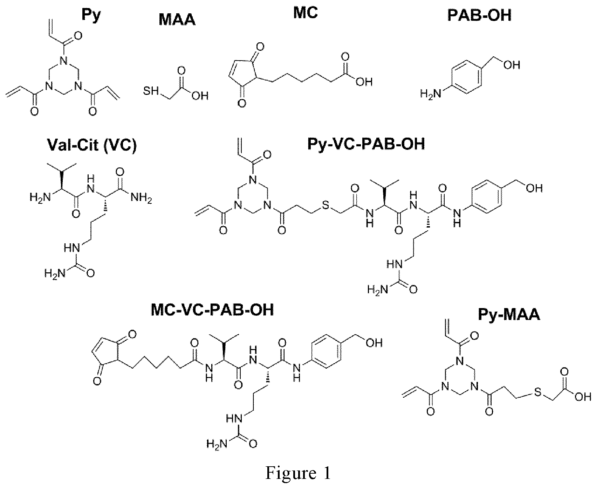

Typically, the linker structure in the ADC of the present invention is shown in FIG. 1.

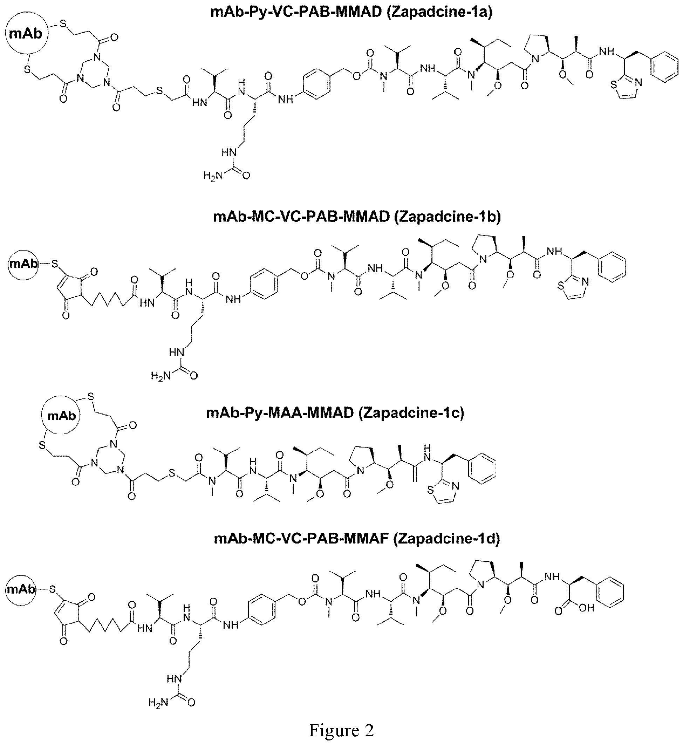

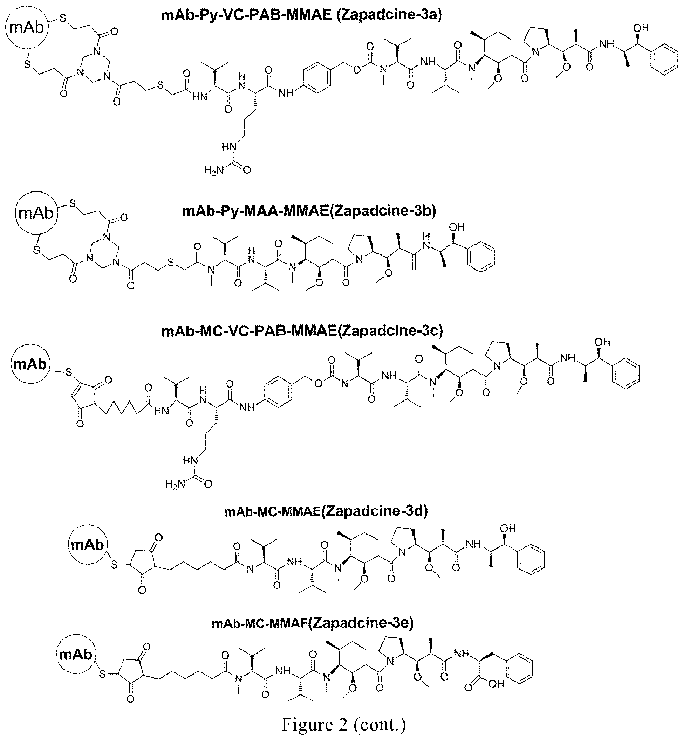

Typically, the structural formulas of the nine ADCs in the present invention are shown in FIG. 2.

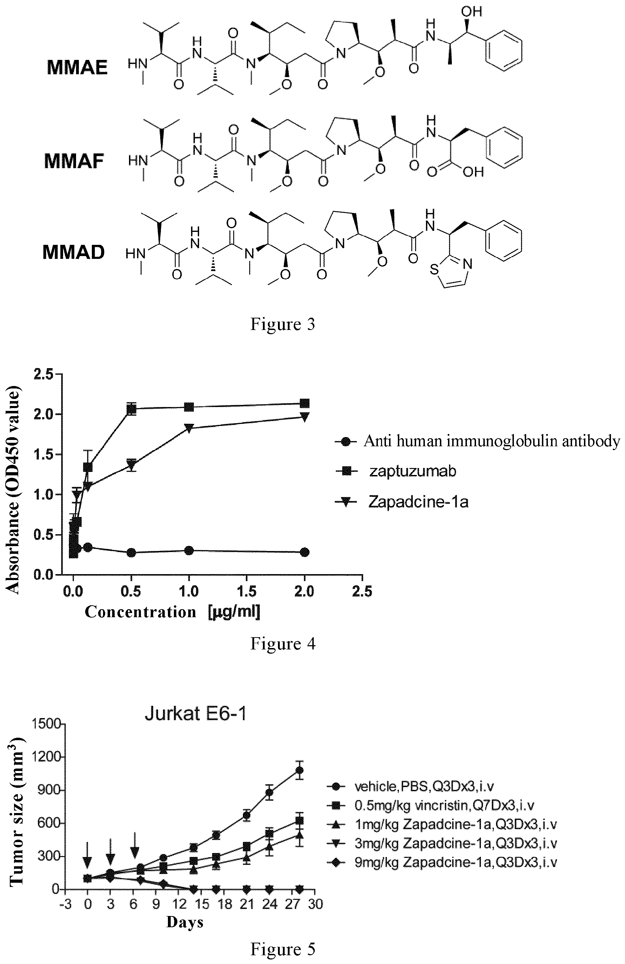

Typically, the structural formula of the drug of the present invention is shown in FIG. 3.

### Application

The present invention further provides use of the antibody according to the present invention, for example, for manufacture of a diagnostic agent, or for manufacture of a medicament for preventing and/or treating a TRAILR2-related disease. The TRAILR2-related disease includes tumorigenesis, tumor growth and/or metastasis, a thrombosis-related disease, inflammation, a metabolism-related disease, etc.

Use of the antibody, ADC or CAR-T according to the present invention includes (but is not limited to):
(i) For diagnosis, prevention and/or treatment of tumorigenesis, tumor growth and/or metastasis, particularly, a solid tumor with TRAILR2 positive expression. The tumors include (but are not limited to): non-small cell lung cancer, liver cancer, colon cancer, breast cancer, ovarian cancer, pancreatic cancer, thyroid cancer, head and neck squamous cell carcinoma, esophageal squamous cell carcinoma, lung squamous cell carcinoma, lung adenocarcinoma, cervical squamous cell carcinoma, pancreatic squamous cell carcinoma, colon squamous cell carcinoma, gastric squamous cell carcinoma, prostate cancer, osteosarcoma and soft tissue sarcoma, more preferably non-small cell lung cancer, liver cancer , ovarian cancer and pancreatic cancer, etc.
(ii) For diagnosis, prevention and/or treatment of tumors in the blood system. The tumors include (but are not limited to): lymphocytic leukemia, myeloid leukemia, non-T and non-B lymphocytic leukemia, follicular lymphoma, mantle cell lymphoma, Burkitt lymphoma, Diffuse large B-cell lymphoma, non-Hodgkin's disease, peripheral T cell lymphoma, angioimmunoblastic T cell lymphoma, anaplastic large cell lymphoma, etc. More preferably, the tumors include acute T lymphocytic leukemia, B lymphocytic leukemia, and non-T and non-B lymphocytic leukemia.

Typically, the present invention relates to the use of Zapadcine-1 (Zapadcine-1a, Zapadcine-1b, Zapadcine-1c, Zapadcine-1d, or a combination thereof) or Zapadcine-3 (Zapadcine-3a, Zapadcine-3b, Zapadcine-3c, Zapadcine-3d, Zapadcine-3e, or a combination thereof) represented by the general formula Ab-(LU-D)p or a pharmaceutically acceptable salt, solvate, stereoisomer, tautomer, prodrug, and a mixture thereof as active ingredients in the preparation of drugs for the prevention and/or treatment of cancer.

### Pharmaceutical composition

The present invention further provides a composition. In the preferred examples, the composition is a pharmaceutical composition comprising the antibody, or an active fragment, a fusion protein or an ADC thereof, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated. The formulated pharmaceutical composition may be administered by conventional routes, including (but not limited to): intratumoral, intraperitoneal, intravenous, or topical administration.

The pharmaceutical composition according to the present invention can be directly used for binding to a TRAILR2 protein molecule, and thus can be used for preventing and treating diseases such as tumors. In addition, other therapeutic agents can be used simultaneously, for example, various cytokines such as TNF, IFN, IL-2, etc.; various chemotherapeutics for tumors, for example, drugs that affect biosynthesis of nucleic acids, such as 5-FU and methotrexate; alkylating agents such as nitrogen mustard and cyclophosphamide; drugs that interfere transcription and block RNA synthesis, such as doxorubicin and actinomycin D; vincristine, camptothecin. Targeting drugs, antibody drugs, inhibitors, for example, anti-PD-1 or PD-L1 antibodies, anti-Fas antibodies, and Bcl-2 inhibitors, etc.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 1 µg per kilogram of body weight to about 5 mg per kilogram of body weight daily. In addition, the polypeptide according to the present invention may also be used in combination with an additional therapeutic agent. When a pharmaceutical composition is used, a safe and effective amount of an immunoconjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, a specific amount should also depend on the factors such as administration route and physical conditions of a patient, which fall into the skills of skilled physicians.

Typically, the present invention provides a pharmaceutical composition comprising a therapeutically effective amount of Zapadcine-1 (Zapadcine-1a, Zapadcine-1b, Zapadcine-1c, Zapadcine- 1d, or a combination thereof) represented by the general formula Ab-(LU-D)p or a pharmaceutically acceptable salt, solvate, stereoisomer, tautomer, prodrug, and pharmaceutically acceptable carriers thereof.

In certain embodiments, the pharmaceutical composition of the present invention uses Zapadcine-1 (Zapadcine-1a, Zapadcine-1b, Zapadcine-1c, or Zapadcine-1d) represented by the general formula mAb-(LU-D)p or a pharmaceutical acceptable salts, ester, solvate, stereoisomer, tautomer, prodrug thereof as active ingredients, used alone or in combination, or formulated with other drugs, excipients, etc. into various dosage forms, including but are not limited to tablets, powders, pills, injections, capsules, films, suppositories, ointments, granules and other forms.

Typically, the present invention provides a pharmaceutical composition comprising a therapeutically effective amount of Zapadcine-3 (Zapadcine-3a, Zapadcine-3b, Zapadcine-3c, Zapadcine- 3d, Zapadcine-3e, or a combination thereof) represented by the general formula Ab-(LU-D)p or a pharmaceutically acceptable salt, solvate, stereoisomer, tautomer, prodrug, and pharmaceutically acceptable carriers thereof.

In certain embodiments, the pharmaceutical composition of the present invention uses Zapadcine-3 (Zapadcine-3a, Zapadcine-3b, Zapadcine-3c, Zapadcine- 3d, Zapadcine-3e) represented by the general formula mAb-(LU-D)p or a pharmaceutical acceptable salts, ester, solvate, stereoisomer, tautomer, prodrug thereof as active ingredients, used alone or in combination, or formulated with other drugs, excipients, etc. into various dosage forms, including but are not limited to tablets, powders, pills, injections, capsules, films, suppositories, ointments, granules and other forms.

### The main advantages of the present invention include:

(1) The ADC of the present invention is a novel, broad-spectrum, high-efficiency, specific anti-tumor anti-TRAILR2 antibody-toxin-conjugate (ADC), which is used for the treatment of TRAILR2-positive tumors and can eradicate the tumor, especially for treatments of lymphocytic leukemia, liver cancer, lung cancer, pancreatic cancer and ovarian cancer, etc..
(2) The therapeutic dose of the ADC of the present invention is low, which greatly improves the drug safety.

The invention is further illustrated below in conjunction with specific embodiments. It should be understood that the examples are not intended to limit the scope of the invention. The experimental methods without detailed conditions in the following examples are generally in accordance with the conditions described in the conventional conditions such as Sambrook. J et al. "Guide to Molecular Cloning Laboratory" (translated by Huang Peitang et al., Beijing: Science Press, 2002), or in accordance with the conditions recommended by the manufacturer (for example, product manuals). Unless otherwise stated, percentages and parts are calculated by weight. Unless otherwise specified, the experimental materials and reagents used in the following examples are commercially available.

In the following, the present invention will be described in more detail with reference to the figures and in combination with examples of the present invention, but the present invention is not limited thereto. The experimental methods in the following examples, unless otherwise specified, are conventional methods; the reagents and biological materials, unless otherwise specified, can be obtained from commercial sources.

### General methods

The technical problem to be solved by the present invention is how to draw lessons from previous clinical trials and to solve the problem of poor curative effect of anti-TRAILR2 monoclonal antibody for tumor treatment. To this end, the inventor adopted the strategy of preparing ADC complex by using the following series of research steps, and ADC candidate drugs of the present invention were obtained. They are:
(1) A CHO original cell bank, a main cell bank and a production cell bank expressing Zaptuzumab were established, and the small-scale process research was completed, in which the expression level in 5 liter bioreactor reached 3.5 g/L.
(2) ¹³¹I-labeled Zaptuzumab was injected through the tail vein of mice, which can specifically target TRAILR2-positive tumors such as lung cancer xenografts.
(3) Using immunocytochemistry technique, it was shown that the fluorescently labeled Zaptuzumab binded to the TRAILR2 receptor on the surface of lung cancer and other tumor cells and can be quickly endocytosed into the lysosome, thus proving that Zaptuzumab has the ability to prepare antibody-toxin-conjugates. The two key features are: the ability of tumor-specific targeting, and the ability to be endocytosed by tumor cells into lysosomes to release small molecule toxins.
(4) Using disulfide bridge coupling technology, Zaptuzumab was coupled with toxins through different chemical linkers, and multiple ADCs with different chemical structures were obtained. After repeated screening of anti-tumor activity *in vivo* and/or *in vitro,* two ADC candidates with excellent druggability (named Zapadcine-1 and Zapadcine-3, respectively) were obtained, which can be used for the treatment of multiple tumors positive for TRAILR2.

### Materials and Methods

### Laboratory animals

BALB/c female mice were purchased from Shanghai Cyper-Bikai Laboratory Animal Co., Ltd. (Shanghai, China) and kept in SPF laboratory animal rooms. NOD-SCID female mice were purchased from Shanghai Lingchang Biotechnology Co., Ltd. (Shanghai, China) and kept in SPF laboratory animal rooms. And the 12 hour light-dark alternate cycle was maintained, with sufficient food and clean drinking water, and the experiment was started at 8 weeks of age. All animal experiments were approved and conducted in accordance with the guidance of the Shanghai Animal Management and Use Committee.

### Cell lines and reagents

Tumor cells such as human lymphocytic leukemia cells, lung cancer cells, liver cancer cells, ovarian cancer cells, pancreatic cancer cells, etc. and normal cells were purchased from the ATCC, or the Cell Center of the Institute of Basic Medical Sciences of the Chinese Academy of Medical Sciences, or the Cell Resource Center of the Shanghai Academy of Life Sciences of Chinese Academy of Sciences, or Saiqi (Shanghai) Bioengineering Co., Ltd., or Saibaikang (Shanghai) Biotechnology Co., Ltd., or Miaotong (Shanghai) Biotechnology Co., Ltd. Paclitaxel, vincristine and epirubicin were all purchased from Selleck. CellTiter-Glo® Luminescence Cell Viability Assay Kit was purchased from Promega.

### Example 1: Zapadcine-1 anti-tumor activity in vitro

Various types of cells with high expression of TRAILR2, such as lymphocyte leukemia cell lines (Jurkat E6-1), lung cancer cell lines (NCI-H460 and NCI-H1975), liver cancer cell lines (SMMC-7221), ovarian cancer cell lines (A2780) and pancreatic cancer Mia PaCa-2, and human normal cell lines or peripheral blood cells, such as human normal peripheral blood mononuclear cells (PBMC), human normal colon epithelial cells (NCM-460), human normal colon tissue cells (CCD-18Co) and human normal lung epithelial cells (BEAS-2B) were used, for evaluating the cytotoxic effects of Zapadcine-1 on various tumor cells and normal cells. The specific research process is as follows: trypsin (0.25%, V/V) was used for digesting the adherent cultured cells (such as NCI-H460 and SMMC-7221, etc.) and the cells were detached, and/or the suspension cultured cells (Jurkat E6-1) were directly collected, then the cells were resuspend in 100 µL complete medium. 5,000 adherent cells or 16000 suspended cells were taken and inoculated in 96-well plates for incubation at 37°C overnight. Then 100µL of medium containing different concentrations of anti-TRAILR2 naked antibody or Zapadcine-1 was added, and placed in the incubator for 72h. Then the cytotoxic effects of test drugs on various tumor cells cultured *in vitro* were determined using the CellTiter-Glo® luciferase activity detection kit (Promega, batch number: 0000217738).

The cell survival rate is calculated by the formula: V_{Sample}/V_{negative} control × 100%. Wherein, Vₛₐₘₚₗₑ is the reading of the drug treatment group, and V_{negative} control is the average value of the solvent control group. Using GraphPad Prism 5.0 software, a non-linear regression model was used to draw the S-type dose-survival curve and the IC₅₀ value was calculated. The IC₅₀ value is obtained by performing a nonlinear fitting calculation on the logarithm value X of the cell concentration (%) to the sample concentration by the following formula.

**Table 4 The cytotoxic effect of Zapadcine-1 of the present invention on various tumor cells and normal cells**

| drug | Zapadcine-1a | | Zapadcine-1b | | Zapadcine-1c | | Zapadcine-1d | | Zaptuzumab | |
|---|---|---|---|---|---|---|---|---|---|---|
| cell | IC50 | eff. | IC50 | eff. | IC50 | eff. | IC50 | eff. | IC50 | eff. |
| SMMC-7721 | 11.98 | 99.43 | 177.7 | 95.72 | 19.73 | 96.49 | 582.7 | 93.66 | 6122 | 47.96 |
| Jurkat E6-1 | 13.35 | 97.31 | 8.71 | 98.62 | 16.46 | 98.14 | 108.2 | 84.93 | 200.9 | 90.71 |
| NCI-H460 | 86.54 | 96.22 | 178.5 | 93.28 | 65.11 | 46.36 | 1026 | 80.61 | NR | NR |
| NCI-H1975 | 109.4 | 84.64 | 410.5 | 83.4 | 311.9 | 83.04 | 1671 | 68.85 | 542.1 | 62.36 |
| A2780 | 151.2 | 96.41 | 1102 | 88.54 | NR | NR | NR | NR | NR | NR |
| Mia PaCa-2 | 135.1 | 88.43 | 604.2 | 86.82 | 173.4 | 74.02 | 1024 | 79.98 | 3007 | 27.52 |
| NCM-460 | NR | NR | NR | NR | NR | NR | NR | NR | NR | NR |
| CCD-18Co | NR | NR | NR | NR | NR | NR | NR | NR | NR | NR |
| BEAS-2B | NR | NR | NR | NR | NR | NR | NR | NR | NR | NR |
| PBMC | NR | NR | NR | NR | NR | NR | NR | NR | NR | NR |

| | | | | | | | | | | |
|---|---|---|---|---|---|---|---|---|---|---|
| Wherein, NR is no response, eff. is efficiency (%), the unit of IC50 is ng/ml | | | | | | | | | | |

The specific results are shown in Table 4. The results show that Zapadcine-1 can inhibit the proliferation of a variety of TRAILR2-positive tumor cells (such as Jurkat E6-1, NCI-H460, NCI-H1975, SMMC-7721, A2780, and Mia PaCa-2, etc.) more effectively than anti-TRAILR2 naked Zaptuzumab, while Zaptuzumab and Zapadcine-1 have no cytotoxic effect on TRAILR2-negative cells (such as human normal PBMC, BEAS-2B, NC-460 and CCD-18Co, etc.).

### Example 2: Affinity between Zapadcine-1a and TRAILR2 detected using ELISA technology

The binding of Zapadcine-1a to the humanized recombinant protein TRAILR2 was evaluated by ELISA. The specific process is as follows: the 96-well plate was coated with 2µg/ml of humanized recombinant protein TRAILR2 with 1×PBS buffer (pH 7.4) in a volume of 100 µl/well, placed at 4°C overnight. The supernatant was discarded and the plate was washed 3 times with PBST (PH 7.4 PBS containing 0.05% Tween 20) buffer, 5 min each time, and added with PBS containing 5% skim milk powder at 240µl/well, incubated at 37°C for 3h, and blocked. The blocking solution was discarded, and the plate was washed 3 times with PBST 300µl/well, 5min each time, added with 50µl/well of the test antibody (primary antibody) or ADC diluted in PBS containing 1% skim milk powder or BSA, in a concentration from 4µg/ml with gradient double-dilution, and with a total of 12 concentrations, triplicated, and incubated at room temperature for 1h. The supernatant was discarded, and the plate was washed 3 times with PBST, 5min each time, added with 50µl/well of the secondary antibody diluted with PBS containing 1% skim milk powder or BSA, at a concentration of 1µg/ml, and incubated at 37°C for 40min. The supernatant was discarded, and the plate was washed 3 times with PBST, 5min each time, added with TMB developer at 50µl/well, and incubated at room temperature for 5-10min. 50µl/well of 1M H₂SO₄ was added according to the color rendering effect to stop the reaction. The OD value at 450nm was read using the SPARK 10M multifunction microplate detector. GraphPad Prism 5.0 software was applied for data analysis. The specific results are shown in Figure 4.

The results show that compared to the humanized monoclonal antibody Zaptuzumab, the affinities of Zapadcine-1a and Zaptuzumab remained at the same order of magnitude, which proves that Zapadcine-1a can effectively bind to TRAILR2 and retains the antigen binding capacity of humanized monoclonal antibody.

### Example 3: Zapadcine-1a inhibits and eliminates subcutaneously transplanted tumors in lymphocytic leukemia mice

The efficacy of Zapadcine-1a against lymphocytic leukemia *in vivo* was evaluated by the growth inhibitory effect of human Jurkat E6-1 leukemia cells with high expression of TRAILR2 in NOD-SCID mice. The specific research process is as follows: a subcutaneous xenograft model of female NOD/SCID mouse (4 weeks old) was established with human T lymphocytic leukemia Jurkat E6-1. Jurkat E6-1 cells were cultured and expanded to a logarithmic growth phase *in vitro.* After the cells counted, they were diluted with PBS buffer into a cell suspension of 1×10⁸/ml. And 0.1ml (1×10⁷ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumor reached 80-100mm³, the tumor-bearing mice were randomly divided into 5 groups, 8 mice in each group, which were the Zapadcine-1a administration groups (low dose group 1mg/kg, medium dose group 3mg/kg, high-dose group 9mg/kg, Q3D×3), negative control group (PBS, Q3D×3) and vincristine positive control group (0.5mg/kg, Q7D×3). The administration time of Zapadcine-1a was 0, 4 and 7 days after grouping, and the administration time of vincristine was 0, 7 and 14 days after grouping, and the above administrations were all via tail vein injection. Tumor sizes (longest diameter and shortest diameter) were measured on days 3, 7, 10, 14, 17, 21, 24, and 28 after administration, respectively. The experiment was ended on the 28th day after the first administration. The animals were immediately euthanized by excessive anesthesia thereafter. The mice were weighed, and tumors were taken out to be weighed and taken pictures, and blood samples were taken for detecting liver and kidney function indexes (ALT, BUN, CERA). The tumor and major tissues and organs (heart, liver, kidney, spleen, lung) were isolated and frozen in liquid nitrogen or fixed with 4% paraformaldehyde to prepare paraffin sections for later use.

The specific results are shown in Figure 5. The results show that on the 14th day after 3 doses of Zapadcine-1a, in the medium and high dose groups (3mg/kg and 9mg/kg), the 100 mm³ lymphocytic leukemia xenograft tumor were completely cleared, and the tumor suppression rate reached 100%. In the low-dose group (1mg/kg), the growth of transplanted tumors can also be significantly inhibited, with a tumor suppression rate of 54.88%. At the end of the experiment on the 28th day, the average tumor weight of the negative control group was 902.1±60.45 mg, while the average tumor weight of the positive control group was 648.7±83.63 mg. The average tumor weight of the Zapadcine-1a low-dose group (1 mg/kg) was 437.9±96.78 mg, and the average tumor weight of Zapadcine-1a medium-dose group (3 mg/kg) and high-dose group (9 mg/kg) was 0 mg.

### Example 4: Zapadcine-1a inhibits human large cell lung cancer cell NCI-H460 subcutaneously transplanted tumor in nude mice

The *in vivo* anti-lung cancer properties of Zapadcine-1a were evaluated by the growth inhibitory effect on human large cell lung cancer cell NCI-H460 with high expression of TRAILR2 in nude mice. The specific research process is as follows: a female BALB/c nude mouse (4 weeks old) subcutaneous tumor model of human NCI-H460 lung cancer cells was established and NCI-H460 cells were cultured and expanded to a logarithmic growth phase *in vitro.* After the cells counted, they were diluted with PBS buffer into a cell suspension of 1×10⁷/ml. And 0.15ml (1×10⁶ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumors reached 80-100mm³, the tumor-bearing mice were randomly divided into 5 groups, 8 mice in each group, which were the Zapadcine-1a administration groups (low dose group 1mg/kg, medium dose group 3mg/kg, high-dose group 9mg/kg, Q3D×3), negative control group (PBS, Q3D×3) and paclitaxel positive control group (10mg/kg, Q3D×3). The administration time of Zapadcine-1a, paclitaxel and negative control group was 0, 4 and 7 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on the 28th day after administration, and the animals were euthanized by overdose anesthesia and weighed immediately thereafter. The tumor tissues were taken out to be weighed and taken pictures. Blood was taken to detect liver and kidney function indexes (ALT, BUN, CERA). The tumor and related organs (heart, liver, kidney, spleen, lung) were isolated and frozen in liquid nitrogen or fixed with 4% paraformaldehyde to prepare paraffin sections for later use.

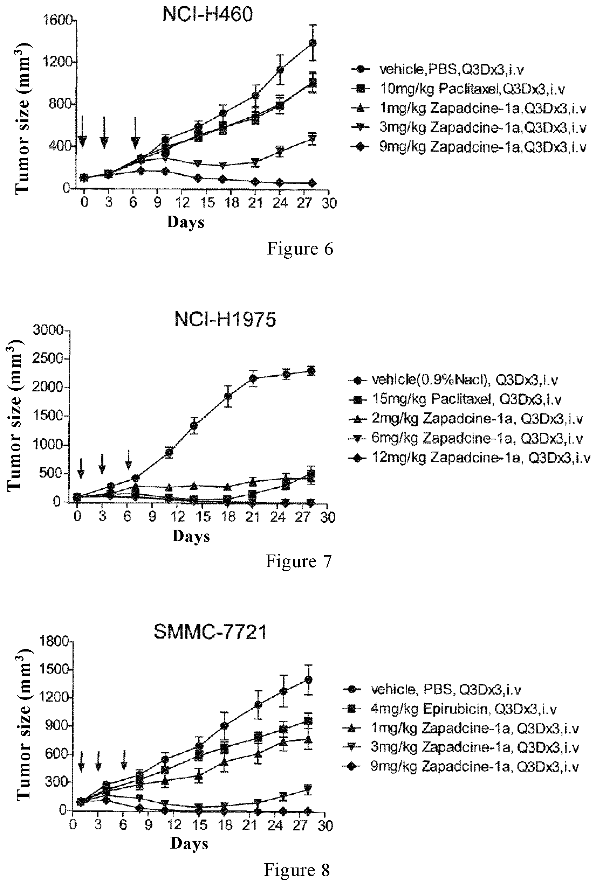

The specific results are shown in Figure 6. The results show that on the 7th day after 3 doses of Zapadcine-1a, the growths of lung cancer xenografts in the high, medium and low dose groups all began to be inhibited. On the 28th day after 3 doses of Zapadcine-1a, the tumor suppression rate in the high-dose group (9 mg/kg) reached 96%, while that in the medium-dose group (3 mg/kg) reached 66%. In the low-dose group (1mg/kg), the growth of transplanted tumors can also be significantly inhibited, with a tumor suppression rate of 28%. At the end of the experiment on the 28th day, the average tumor weight of the negative control group was 1.47±0.45g, while the average tumor weight of the positive control group was 1.13±0.42g. The average tumor weight of the Zapadcine-1a low-dose group (1 mg/kg) was 0.99±0.26g, and the average tumor weight of Zapadcine-1a medium-dose group (3 mg/kg) was 0.55±0.21g, and that of high-dose group (9 mg/kg) was 0.08±0.07g.

### Example 5: Zapadcine-1a inhibits human non-small cell lung cancer cell NCI-H1975 xenograft in nude mice

The *in vivo* anti-human non-small cell lung cancer properties of Zapadcine-1a were evaluated by the inhibitory effect on the xenografts of human non-small cell lung cancer cells NCI-H1975 with high expression of TRAILR2 in nude mice. The specific research process is as follows: a female BALB/c nude mouse (4 weeks old) subcutaneously transplanted tumor model of human NCI-H1975 lung cancer cells was established. NCI-H1975 cells were cultured and expanded to a logarithmic growth phase *in vitro.* After the cells counted, they were diluted with PBS buffer into a cell suspension of 1×10⁷/ml. And 0.15ml (1.5×10⁶ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumors reached 80-100mm³, the tumor-bearing mice were randomly divided into 5 groups, 8 mice in each group, which were the Zapadcine-1a administration groups (low dose group 1mg/kg, medium dose group 3mg/kg, high-dose group 9mg/kg, Q3D×3), negative control group (PBS, Q3D×3) and paclitaxel positive control group (10mg/kg, Q3D×3). The administration time of Zapadcine-1a, paclitaxel positive control group and negative control group was 0, 4 and 7 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on day 28 after administration, and the mice were euthanized by overdose anesthesia and weighed immediately thereafter. The tumor tissues were taken out to be weighed and taken pictures. Blood was taken to detect liver and kidney function indexes (ALT, BUN, CERA). The tumor and related organs (heart, liver, kidney, spleen, lung) were isolated and frozen in liquid nitrogen or fixed with 4% paraformaldehyde to prepare paraffin sections for later use.

The specific results are shown in Figure 7. The results show that on the 7th day after 3 doses of Zapadcine-1a, the growths of lung cancer xenografts in the high, medium and low dose groups all began to be inhibited. On the 21st day after 3 doses of Zapadcine-1a, the tumor suppression rate in the medium and high dose groups (6 mg/kg and 12 mg/kg) both reached 100%, while in the low dose group (2 mg/kg), the transplanted tumor growth was also significantly inhibited, and the tumor suppression rate reached 84%. At the end of the experiment on the 28th day, the average tumor weight of the negative control group was 2.09±0.08g, while the average tumor weight of the positive control group was 0.58±0.15g. The average tumor weight of the Zapadcine-1a low-dose group (2mg/kg) was 0.58±0.17g, and the average tumor weight of Zapadcine-1a medium-dose group (6mg/kg) was 0.01±0.01g, and that of high-dose group (12mg/kg) was 0.0g.

### Example 6: Zapadcine-1a inhibits the liver cancer cell SMMC-7721 xenograft in nude mice

The *in vivo* anti-liver cancer properties of Zapadcine-1a were evaluated by the growth inhibitory effect on human liver cancer cell SMMC-7721 with positively expression of TRAILR2 in nude mice. The specific research process is as follows: a female BALB/c nude mouse (4 weeks old) subcutaneously transplanted tumor model of human SMMC-7721 liver cancer cells was established and SMMC-7721 cells were cultured and expanded to a logarithmic growth phase *in vitro.* After the cells counted, they were diluted with PBS buffer into a cell suspension of 1×10⁷/ml. And 0.15 ml (1.5×10⁶ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumors reached 80-100mm³, the tumor-bearing mice were randomly divided into 5 groups, 8 mice in each group, which were the Zapadcine-1a administration groups (low dose group 1mg/kg, medium dose group 3mg/kg, high-dose group 9mg/kg, Q3D×3), negative control group (PBS, Q3D×3) and epirubicin positive control group (5mg/kg, Q3D×9). The administration time of Zapadcine-1a and negative control group was 0, 4 and 7 days after grouping, and the administration time of epirubicin was 0, 4, 7, 10, 14, 17 and 21 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on day 28 after administration, and the mice were euthanized by overdose anesthesia and weighed immediately thereafter. The tumor tissues were taken out to be weighed and taken pictures. Blood was taken to detect liver and kidney function indexes (ALT, BUN, CERA). The tumor tissues and major organs (heart, liver, kidney, spleen, lung) were isolated and frozen in liquid nitrogen or fixed with 4% paraformaldehyde to prepare paraffin sections for later use.

Specific results are shown in Figure 8. The results of the study show that on the 7th day after 3 doses of Zapadcine-1a, the growths of liver cancer xenografts in the high, medium and low dose groups all began to be inhibited. On the 11th day after 3 doses of Zapadcine-1a, the tumor suppression rate in the high-dose group (9 mg/kg) reached 100%, while that in the medium-dose group (3 mg/kg) reached 89%. In the low-dose group (1mg/kg), the growth of transplanted tumors can also be significantly inhibited, with a tumor suppression rate of 48%. At the end of the experiment on the 28th day, the average tumor weight of the negative control group was 1.37±0.17g, while the average tumor weight of the positive control group was 1.02±0.08g. The average tumor weight of the Zapadcine-1a low-dose group (1 mg/kg) was 0.88±0.12g, and the average tumor weight of Zapadcine-1a medium-dose group (3 mg/kg) was 0.26±0.07g, and that of high-dose group (9 mg/kg) was 0g.

### Example 7: Zapadcine-1a inhibits ovarian cancer cell A2780 xenograft in nude mice

The *in vivo* anti-ovarian cancer properties of Zapadcine-1a were evaluated by the growth inhibitory effect on human ovarian cancer cell A2780 with positively expression of TRAILR2 in nude mice. The specific research process is as follows: a female BALB/c nude mouse (4 weeks old) subcutaneously transplanted tumor model of human A2780 ovarian cancer cells was established and A2780 ovarian cells were cultured and expanded to a logarithmic growth phase *in vitro.* After the cells counted, they were diluted with PBS buffer into a cell suspension of 1×10⁷/ml. And 0.15 ml (1.5×10⁶ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumors reached 80-100mm³, the tumor-bearing mice were randomly divided into 5 groups, 8 mice in each group, which were the Zapadcine-1a administration groups (low dose group 1 mg/kg, medium dose group 3 mg/kg, high-dose group 9 mg/kg, Q3D×3), negative control group (PBS, Q3D×3) and paclitaxel positive control group (10mg/kg, Q3D×3). The administration time of Zapadcine-1a, paclitaxel and negative control group was 0, 4 and 7 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on day 28 after administration, and the mice were euthanized by overdose and weighed immediately thereafter. The tumor tissues were taken out to be weighed and taken pictures. Blood was taken to detect liver and kidney function indexes (ALT, BUN, CERA). The tumor tissues and major organs (heart, liver, kidney, spleen, lung) were isolated and frozen in liquid nitrogen or fixed with 4% paraformaldehyde to prepare paraffin sections for later use.

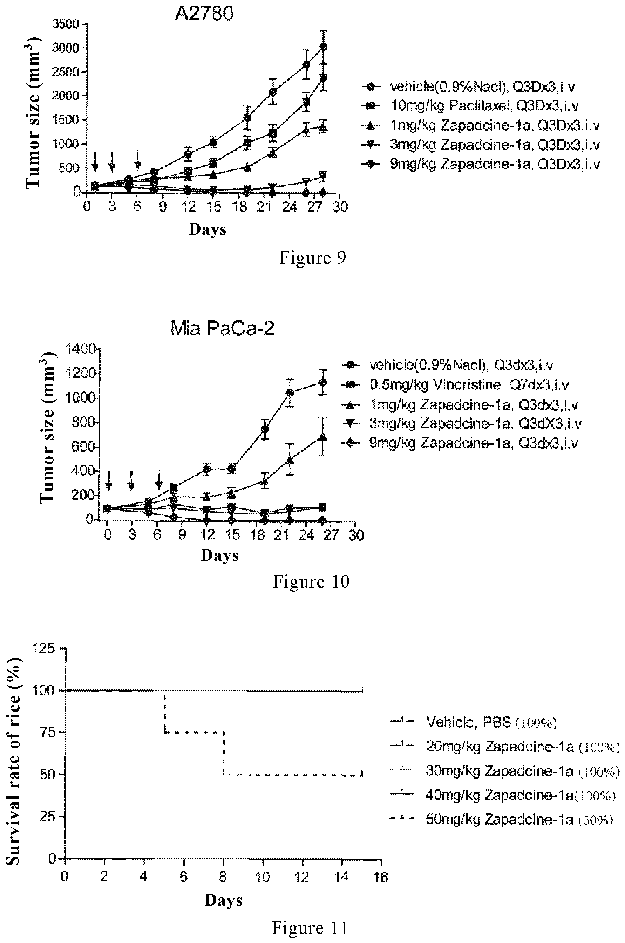

The specific results are shown in Figure 9. The results of the study show that on the 7th day after 3 doses of Zapadcine-1a, the growths of ovarian cancer xenografts in the high, medium and low dose groups all began to be inhibited. On the 18th day after 3 doses of Zapadcine-1a, the tumor suppression rate in the high-dose group (9 mg/kg) reached 100%, while that in the medium-dose group (3 mg/kg) reached 92%. In the low-dose group (1mg/kg), the growth of transplanted tumors can also be significantly inhibited, with a tumor suppression rate of 56%. At the end of the experiment on the 28th day, the average tumor weight of the negative control group was 2.88±0.34g, while the average tumor weight of the positive control group was 2.42±0.35g. The average tumor weight of the Zapadcine-1a low-dose group (1mg/kg) was 1.40±0.15g, and the average tumor weight of Zapadcine-1a medium-dose group (3mg/kg) was 0.27±0.10g, and that of high-dose group (9mg/kg) was 0g.

### Example 8: Zapadcine-1a inhibits pancreatic cancer cell Mia PaCa-2 xenograft in nude mice

The *in vivo* anti-ovarian cancer properties of Zapadcine-1a were evaluated by the growth inhibitory effect on human Mia PaCa-2 pancreatic cancer cell with positively expression of TRAILR2 in nude mice. The specific research process is as follows: a female BALB/c nude mouse (4 weeks old) subcutaneously transplanted tumor model of human Mia PaCa-2 pancreatic cancer cells was established and Mia PaCa-2 pancreatic cancer cells were cultured and expanded to a logarithmic growth phase *in vitro.* After the cells counted, they were diluted with PBS buffer into a cell suspension of 1×10⁷/ml. And 0.15 ml (1.5×10⁶ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumors reached 80-100mm³, the tumor-bearing mice were randomly divided into 5 groups, 8 mice in each group, which were the Zapadcine-1a administration groups (low dose group 1 mg/kg, medium dose group 3 mg/kg, high-dose group 9 mg/kg, Q3D×3), negative control group (PBS, Q3D×3) and paclitaxel positive control group (10mg/kg, Q3D×3). The administration time of Zapadcine-1a, paclitaxel and negative control group was 0, 4 and 7 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on day 28 after administration, and the mice were euthanized by overdose and weighed immediately thereafter. The tumor tissues were taken out to be weighed and taken pictures. Blood was taken to detect liver and kidney function indexes (ALT, BUN, CERA). The tumor tissues and major organs (heart, liver, kidney, spleen, lung) were isolated and frozen in liquid nitrogen or fixed with 4% paraformaldehyde to prepare paraffin sections for later use.

The specific results are shown in Figure 10. The results of the study show that on the 7th day after 3 doses of Zapadcine-1a, the growths of pancreatic cancer xenografts in the high, medium and low dose groups all began to be inhibited. On the 12th day after 3 doses of Zapadcine-1a, the tumor suppression rate in the high-dose group (9 mg/kg) reached 100%, while that in the medium-dose group (3 mg/kg) reached 91%. In the low-dose group (1mg/kg), the growth of transplanted tumors can also be significantly inhibited, with a tumor suppression rate of 39%.

### Example 9: Acute toxic effect of Zapadcine-1a on normal mice

The acute toxicity of Zapadcine-1 was evaluated by examining the changes in the physical state and biochemical indexes of normal mice after administration. The specific research process is as follows: 20 healthy BALB/c female mice (purchased from Shanghai Xipuer-Bikai Experimental Animal Co., Ltd.) were taken, which were 6-7 weeks old after birth, with a weight of 18-22g. They were divided into 5 groups, 4 mice in each group. After a week of regular feeding, the drug administration began. The dosages of Zapadcine-1a was 20 mg/kg, 30 mg/kg, 40 mg/kg and 50 mg/kg, and the blank solvent was used as a normal control. Single administration was conducted by slow injection through the tail vein, and the situation of mice including death, diet and exercise were observed every day. The weight of mice was recorded every 3 days on time, and blood samples were taken for blood biochemical detection on days 5 and 15, respectively. Test items include ALT and UREA. On the 15th day after administration, the animals were euthanized by overdose anesthesia.

The specific results are shown in Figure 11. The results show that compared with the control group, the mice in the three dose groups of Zapadcine-1a of 20 mg/kg, 30 mg/kg and 40 mg/kg grew normally, exercised well, had normal weight changes, had normal liver and kidney functions, and none of them died. However, the mortality rate of mice in the Zapadcine-1a 50 mg/kg dose group reached 50%. The body weights in the early stage became smaller, and the growths of the mice in the later stage returned to normal. The body weights changed in the mice were normal, and the liver and kidney functions of the mice were normal. It indicates that Zapadcine-1a has a good safety, and the maximum tolerated single dose of Zapadcine-1a in mice is 40-50mg/kg.

### Example 10: Zapadcine-3 anti-tumor activity in vitro

Various types of cells with high expression of TRAILR2, such as lymphocyte leukemia cell lines (Jurkat E6-1, Reh), lung cancer cell lines (MSTO-211H), glioma cell lines (A172) and pancreatic cancer Mia PaCa-2, and human normal cell lines or peripheral blood cells, such as human normal peripheral blood mononuclear cells (PBMC), human normal colon epithelial cells (NCM-460), human normal colon tissue cells (CCD-18Co) and human normal lung epithelial cells (BEAS-2B) were used, for evaluating the cytotoxic effects of Zapadcine-3 on various tumor cells and normal cells. The specific research process is as follows: trypsin (0.25%, V/V) was used for digesting the adherent cultured cells (such as MSTO-211H, etc.) and the cells were detached, and/or the suspension cultured cells (Jurkat E6-1) were directly collected, then the cells were resuspend in 100 µL complete medium. 5,000 adherent cells or 16000 suspended cells were taken and inoculated in 96-well plates for incubation at 37°C overnight. Then 100µL of medium containing different concentrations of anti-TRAILR2 naked antibody or Zapadcine-3 was added, and placed in the incubator for 72h. Then the cytotoxic effects of test drugs on various tumor cells cultured *in vitro* were determined using the CellTiter-Glo® luciferase activity detection kit (Promega, batch number: 0000217738).

The cell survival rate is calculated by the formula: Vₛₐₘₚₗₑ/V_{negative} control × 100%. Wherein, Vₛₐₘₚₗₑ is the reading of the drug treatment group, and V_{negative} control is the average value of the solvent control group. Using GraphPad Prism 5.0 software, a non-linear regression model was used to draw the S-type dose-survival curve and the IC₅₀ value was calculated. The IC₅₀ value is obtained by performing a nonlinear fitting calculation on the logarithm value X of the cell concentration (%) to the sample concentration by the following formula.

**Table 5 The cytotoxic effect of Zapadcine-3 of the present invention on various tumor cells and normal cells**

| drug | Zapadcine-3a | | Zapadcine-3b | | Zapadcine-3 c | | Zapadcine-3d | | Zapadcine-3e | | Zaptuzumab | |
|---|---|---|---|---|---|---|---|---|---|---|---|---|
| cell | IC50 | eff. | IC50 | eff. | IC50 | eff. | IC50 | eff. | IC50 | eff. | IC50 | eff. |
| Jurkat E6-1 | 37.15 | 80.16 | 71.65 | 87.13 | 89.57 | 81.96 | 5.705 | 98.88 | 98.06 | 90.66 | 200.9 | 90.71 |
| Reh | 15.93 | 89.81 | 195.5 | 75.26 | 889.4 | 98.59 | 24.18 | 98.78 | 162.9 | 77.38 | 1591 | 45.01 |
| MSTO-211H | 127.1 | 97.53 | 255.1 | 90.98 | 219.9 | 98.21 | 123.5 | 98.45 | 177.5 | 99.11 | 514.2 | 47.82 |
| A172 | 225.3 | 98.23 | 388.8 | 90.83 | 703.3 | 93.21 | 307.5 | 92.48 | NR | NR | NR | NR |
| Mia PaCa-2 | 97.03 | 88.78 | 414.7 | 52.92 | 508.1 | 86.31 | 139.3 | 81.66 | 951.2 | 52.95 | 2899 | 33.25 |
| NCM-460 | NR | NR | NR | NR | NR | NR | NR | NR | NR | NR | NR | NR |
| CCD-18Co | NR | NR | NR | NR | NR | NR | NR | NR | NR | NR | NR | NR |
| BEAS-2B | NR | NR | NR | NR | NR | NR | NR | NR | NR | NR | NR | NR |
| PBMC | NR | NR | NR | NR | NR | NR | NR | NR | NR | NR | NR | NR |

| | | | | | | | | | | | | |
|---|---|---|---|---|---|---|---|---|---|---|---|---|
| Wherein, NR is no response, eff. is efficiency (%), the unit of IC50 is ng/ml | | | | | | | | | | | | |

The specific results are shown in Table 5. The results show that Zapadcine-3 (such as especially Zapadcine-3a and Zapadcine-3d) can inhibit the proliferation of a variety of TRAILR2-positive tumor cells (such as Jurkat E6-1, Reh, MSTO-211H, A172 and Mia PaCa-2, etc.) more effectively than anti-TRAILR2 naked Zaptuzumab, while Zaptuzumab and Zapadcine-3 have no cytotoxic effect on TRAILR2-negative cells (such as human normal PBMC, BEAS-2B, NCM-460 and CCD-18Co, etc.).

### Example 11: Affinity between Zapadcine-3a and TRAILR2 detected using ELISA technology

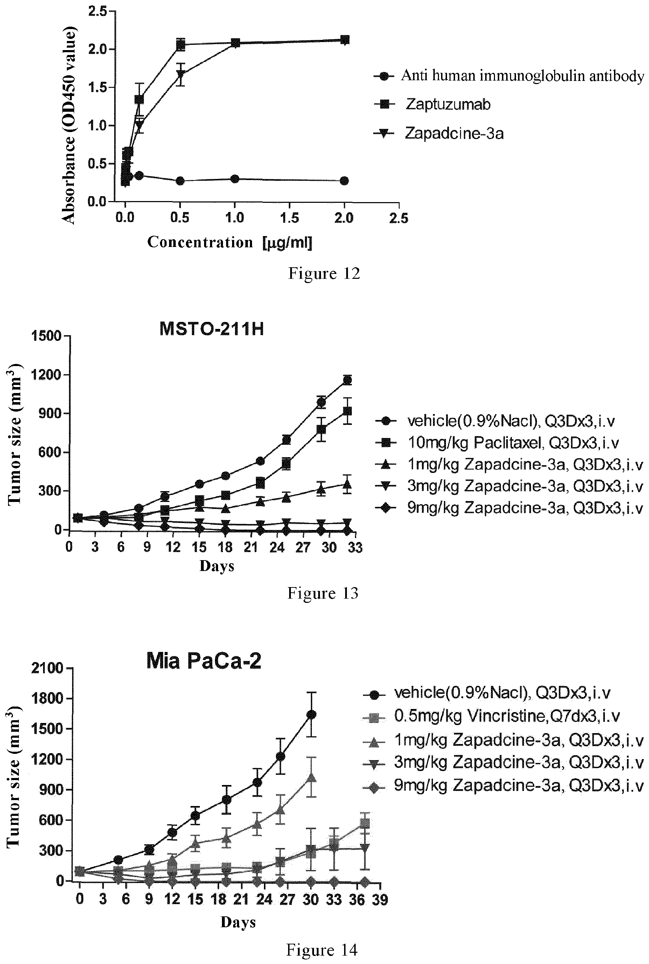

The binding of Zapadcine-3a to the humanized recombinant protein TRAILR2 was evaluated by ELISA. The specific process is as follows: the 96-well plate was coated with 2µg/ml of humanized recombinant protein TRAILR2 with 1×PBS buffer (pH 7.4) in a volume of 100 µl/well, placed at 4°C overnight. The supernatant was discarded and the plate was washed 3 times with PBST (PH 7.4 PBS containing 0.05% Tween 20) buffer, 5 min each time, and added with PBS containing 5% skim milk powder at 240µl/well, incubated at 37°C for 3h, and blocked. The blocking solution was discarded, and the plate was washed 3 times with PBST 300µl/well, 5min each time, added with 50µl/well of the test antibody (primary antibody) or ADC diluted in PBS containing 1% skim milk powder or BSA, in a concentration from 4µg/ml with gradient double-dilution, and with a total of 12 concentrations, triplicated, and incubated at room temperature for 1h. The supernatant was discarded, and the plate was washed 3 times with PBST, 5min each time, added with 50µl/well of the secondary antibody diluted with PBS containing 1% skim milk powder or BSA, at a concentration of 1µg/ml, and incubated at 37°C for 40min. The supernatant was discarded, and the plate was washed 3 times with PBST, 5min each time, added with TMB developer at 50µl/well, and incubated at room temperature for 5-10min. 50µl/well of 1M H₂SO₄ was added according to the color rendering effect to stop the reaction. The OD value at 450nm was read using the SPARK 10M multifunction microplate detector. GraphPad Prism 5.0 software was applied for data analysis. The specific results are shown in Figure 12.

The results show that compared to the humanized monoclonal antibody Zaptuzumab, the affinities of Zapadcine-3a and Zaptuzumab remained at the same order of magnitude, which proves that Zapadcine-3a can effectively bind to TRAILR2 and retains the antigen binding capacity of humanized monoclonal antibody.

### Example 12: Zapadcine-3a inhibits human non-small cell lung cancer cell MSTO-211H xenograft in nude mice

The *in vivo* anti-human non-small cell lung cancer properties of Zapadcine-3a were evaluated by the inhibitory effect on the xenografts of human non-small cell lung cancer cells MSTO-211H with high expression of TRAILR2 in nude mice. The specific research process is as follows: a female BALB/c nude mouse (4 weeks old) subcutaneously transplanted tumor model of human MSTO-211H lung cancer cells was established. MSTO-211H cells were cultured and expanded to a logarithmic growth phase *in vitro.* After the cells counted, they were diluted with PBS buffer into a cell suspension of 1×10⁷/ml. And 0.15ml (1.5×10⁶ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumors reached 80-100mm³, the tumor-bearing mice were randomly divided into 5 groups, 8 in each group, which were the Zapadcine-3a administration groups (low dose group 1mg/kg, medium dose group 3mg/kg, high-dose group 9mg/kg, Q3D×3), negative control group (PBS, Q3D×3) and paclitaxel positive control group (10mg/kg, Q3D×3). The administration time of Zapadcine-3a, paclitaxel positive control group and negative control group was 0, 4 and 7 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on day 32 after administration, and the mice were euthanized by overdose anesthesia and weighed immediately thereafter. The tumor tissues were taken out to be weighed and taken pictures. Blood was taken to detect liver and kidney function indexes (ALT, BUN, CERA). The tumor and related organs (heart, liver, kidney, spleen, lung) were isolated and frozen in liquid nitrogen or fixed with 4% paraformaldehyde to prepare paraffin sections for later use.

The specific results are shown in Figure 13. The results show that on the 7th day after 3 doses of Zapadcine-3a, the growths of lung cancer xenografts in the high, medium and low dose groups all began to be inhibited. On the 18th day after 3 doses of Zapadcine-3a, the tumor suppression rate in the high dose group (9mg/kg) reached 100%, while in the low dose group (1 and 3 mg/kg), the transplanted tumor growth was also significantly inhibited, and the tumor suppression rates reached 65% and 93%, respectively.

### Example 13: Zapadcine-3a inhibits pancreatic cancer cell Mia PaCa-2 xenograft in nude mice

The *in vivo* anti-ovarian cancer properties of Zapadcine-3a were evaluated by the growth inhibitory effect on human Mia PaCa-2 pancreatic cancer cells with positively expression of TRAILR2 in nude mice. The specific research process is as follows: a female BALB/c nude mouse (4 weeks old) subcutaneously transplanted tumor model of human Mia PaCa-2 pancreatic cancer cells was established and Mia PaCa-2 pancreatic cancer cells were cultured and expanded to a logarithmic growth phase *in vitro.* After the cells counted, they were diluted with PBS buffer into a cell suspension of 1×10⁷/ml. And 0.15 ml (1.5×10⁶ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumor reached 80-100mm³, the tumor-bearing mice were randomly divided into 5 groups, 8 mice in each group, which were the Zapadcine-3a administration groups (low dose group 1 mg/kg, medium dose group 3 mg/kg, high dose group 9 mg/kg, Q3D×3), negative control group (PBS, Q3D×3) and vincristine positive control group (10mg/kg, Q7D×3). The administration time of Zapadcine-3a and negative control group was 0, 4 and 7 days after grouping, and the administration time of vincristine was 0, 7 and 14 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on day 28 after administration, and the mice were euthanized by overdose anesthesia and weighed immediately thereafter. The tumor tissues were taken out to be weighed and taken pictures. Blood was taken to detect liver and kidney function indexes (ALT, BUN, CERA). The tumor tissues and major organs (heart, liver, kidney, spleen, lung) were isolated and frozen in liquid nitrogen or fixed with 4% paraformaldehyde to prepare paraffin sections for later use.

The specific results are shown in Figure 14. The results of the study show that on the 5th day after 2 doses of Zapadcine-3a, the growths of pancreatic cancer xenografts in the high, medium and low dose groups all began to be inhibited. On the 9th day after 3 doses of Zapadcine-3a, the tumor suppression rate in the high-dose group (9 mg/kg) reached 100%, while that in the medium-dose group (3 mg/kg) reached 91%. In the low-dose group (1mg/kg), the growth of transplanted tumors can also be significantly inhibited, with a tumor suppression rate of 45%.

### Example 14: The inhibitory effect of Zapadcine-3a on T lymphocyte leukemia cell Jurkat E6-1 xenografts in nude mice.

The *in vivo* anti-ovarian cancer properties of Zapadcine-3a were evaluated by the growth inhibitory effect on human T lymphocyte leukemia cell Jurkat E6-1 with positively expression of TRAILR2 in nude mice. The specific process is as follows: a female BALB/c nude mouse (5-6 weeks old) subcutaneously transplanted tumor model of human Jurkat E6-1 was established and Jurkat E6-1 cells were cultured and expanded to a logarithmic growth phase *in vitro.* After the cells counted, they were diluted with PBS buffer into a cell suspension of 1×10⁸/ml. And 0.1 ml (1×10⁷ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumors reached 80-100mm³, the tumor-bearing mice were randomly divided into 4 groups, 8 mice in each group, which were the Zapadcine-3a administration groups (low dose group 1 mg/kg, medium dose group 3 mg/kg, high-dose group 9 mg/kg, Q3D×3), negative control group (PBS, Q3D×3). The administration time of Zapadcine-3a and negative control group was 0, 4 and 7 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on day 28 after administration, and the mice were euthanized by overdose anesthesia and weighed immediately thereafter. The tumor tissues were taken out to be weighed and taken pictures. The tumor tissues and major organs (heart, liver, kidney, spleen, lung) were isolated and frozen in liquid nitrogen or fixed with 4% paraformaldehyde to prepare paraffin sections for later use.

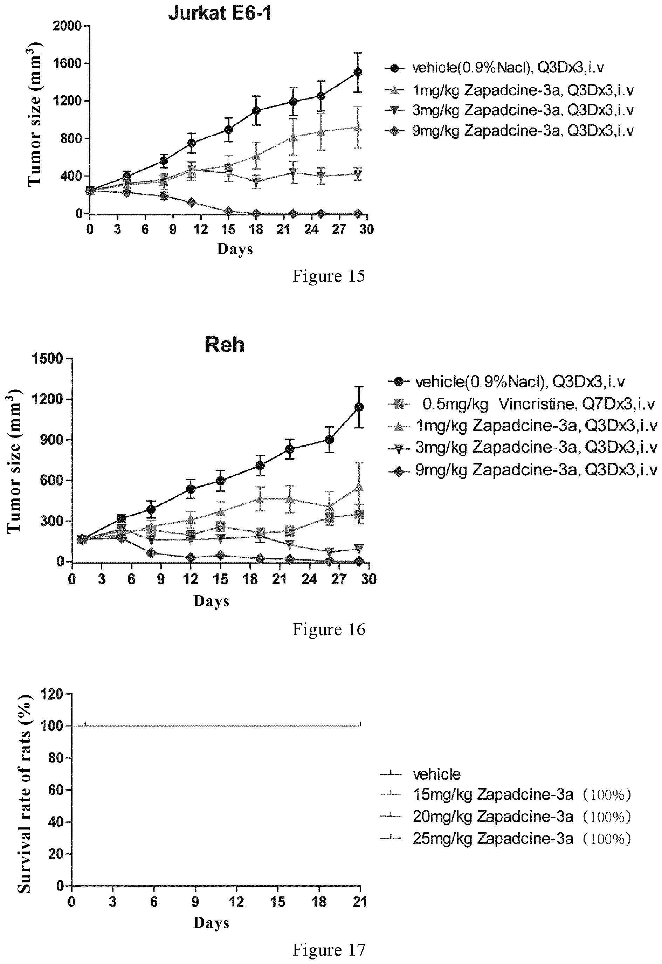

The specific results are shown in Figure 15. The results show that in the Jurkat E6-1 mouse subcutaneous xenograft model, on the 14th day after 3 doses of Zapadcine-3a, in the high dose group (9mg/kg), the 100 mm³ lymphocytic leukemia xenograft tumor were completely cleared, and the tumor suppression rate reached 100%. In the medium dose group (3mg/kg), the growth of transplanted tumors can also be significantly inhibited, with a tumor suppression rate of 84.88%.

### Example 15: The inhibitory effect of Zapadcine-3a on non-T and non-B lymphocyte leukemia cell Reh xenografts in nude mice.

The *in vivo* anti-ovarian cancer properties of Zapadcine-3a were evaluated by the growth inhibitory effect on human non-T and non-B lymphocyte leukemia cell Reh with positively expression of TRAILR2 in nude mice. The specific research process is as follows: a female BALB/c nude mouse (5 weeks old) subcutaneously transplanted tumor model of human Reh cells was established and Reh cells were cultured and expanded to a logarithmic growth phase *in vitro.* After the cells counted, they were diluted with PBS buffer into a cell suspension of 1×10⁸/ml. And 0.1 ml (1×10⁷ cells) of the cell suspension was taken and inoculated subcutaneously on the right back of mice with a 1ml syringe. The skin at the inoculation site was sterilized with 70% alcohol, and the survival status of the animals was observed and the growth of transplanted tumor was measured and recorded regularly after inoculation. When the average size of the tumor reached 80-100mm³, the tumor-bearing mice were randomly divided into 5 groups, 8 mice in each group, which were the Zapadcine-3a administration groups (low dose group 1 mg/kg, medium dose group 3 mg/kg, high dose group 9 mg/kg, Q3D×3), negative control group (PBS, Q3D×3) and vincristine positive control group (0.5mg/kg, Q7D×3). The administration time of Zapadcine-3a and negative control group was 0, 4 and 7 days after grouping, and the administration time of vincristine was 0, 7 and 14 days after grouping, and the above administrations were all via tail vein injection. Tumor sizes (longest diameter and shortest diameter) were measured on days 4, 7, 10, 14, 17, 21, 24, and 28 after administration, respectively. And the experiment was terminated on day 28 after administration, and the mice were euthanized by overdose anesthesia and weighed immediately thereafter. The tumor tissues were taken out to be weighed and taken pictures. The tumor tissues and major organs (heart, liver, kidney, spleen, lung) were isolated and frozen in liquid nitrogen or fixed with 4% paraformaldehyde to prepare paraffin sections for later use.

The specific results are shown in Figure 16. The results show that in the Reh mouse subcutaneous xenograft model, on the 21st day after 3 doses of Zapadcine-3a, in the high dose group (9mg/kg), the 100 mm³ lymphocytic leukemia xenograft tumor were completely cleared, and the tumor suppression rate reached 100%. In the medium dose group (3mg/kg), the growth of transplanted tumors can also be significantly inhibited, with a tumor suppression rate of 93.28%.

### Example 16: Acute toxic effect of Zapadcine-3a on normal rats

The acute toxicity of Zapadcine-3a was evaluated by examining the changes in the physical state and biochemical indexes of normal rats after administration. The specific research process is as follows: 20 healthy SD rats (purchased from Shanghai Xipuer-Bikai Experimental Animal Co., Ltd.) were taken, which were 5-6 weeks old after birth, with a weight of 160-180g. They were divided into 4 groups, 5 rats in each group. After a week of regular feeding, the drug administration began. The dosages of Zapadcine-3a was 15 mg/kg, 20 mg/kg and 25mg/kg, and the blank solvent was used as a normal control. Single administration was conducted by slow injection through the tail vein, and the situation of rats including death, diet and exercise were observed every day. The weight of rats was recorded every 3 days on time, and blood samples were taken for blood biochemical detection on days 5 and 15, respectively. Test items include ALT and UREA. On the 21st day after administration, the animals were euthanized by overdose anesthesia.

The specific results are shown in Figure 17. The results show that compared with the control group, the rats in the two dose groups of Zapadcine-3a of 15 mg/kg and 20 mg/kg grew normally, exercised well, had normal weight changes, had normal liver and kidney functions, and none of them died. The rats in the Zapadcine-3a 25 mg/kg dose group grew normally and all survived, but the changes in the body weight of the rats slowed down, indicating that Zapadcine-3 a has a good safety, and the maximum tolerated single dose of Zapadcine-3a in rats is no less than 25mg/kg.

### Example 17: Acute toxic effect of Zapadcine-3a on normal cynomolgus monkeys

The acute toxicity of Zapadcine-3a was evaluated by examining the changes in the physical state and biochemical indexes of normal cynomolgus monkeys after administration. The specific research process is as follows: 3 female Non-Naive cynomolgus monkeys were selected, and the animal weights were 3.19-3.63 kg. Drugs were administered by intravenous infusion, with a dosage of 2, 3, 4 mg/kg, and an administration volume of 2 mL/kg. Animals were administered once on the dosing day, and continuously observed for 21 days thereafter. The animals were weighed on D-1, before D1 administration, on D8, D15, D22 (before dissection). And the food consumptions of animals within 24 hours (24 hr ± 1 hr) on D2, D8, D15, D21 were determined. Hematology, blood coagulation, blood biochemical test and urine analysis were performed before administration and on the 22nd day after administration. Before administration, and 20min, 1h, 2h, 4h, 8h, 24h, 48h, 72h, 96h, 168h, 240h, 336h, 504h after administration, EDTA-K2 anticoagulated plasma samples were collected for plasma drug concentration detection. Animals were euthanized at D22, and liver, spleen, kidney, heart, lung and other tissues were collected and stored, and the liver, spleen, kidney, and heart were weighed.

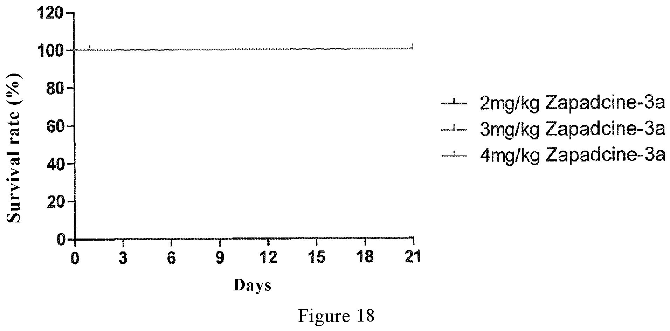

The specific results are shown in Figure 18. The results show that when a single intravenous infusion of compound Zapadcine-3a was administered to female cynomolgus monkey, with a dosage of 2, 3, and 4 mg/kg, respectively, and after continuous observation for 21 days, there was no obvious abnormality in animal weight and food consumption. D22 after drug administration, the reticulocyte count and reticulocyte percentage in hematological data were significantly higher than those before administration, which presumably related to the drug effect. The CK (creatine kinase) values of animals in each administration group showed a downward trend compared with those before administration. There was no obvious abnormality in the blood coagulation index, urine index and organ weight data of each group of animals. Under the conditions of this experiment, when a female cynomolgus monkey was given intravenous infusion of 2, 3, and 4 mg/kg of Zapadcine-3a, the maximum tolerated dose was 4 mg/kg.

### Discussion

In ADC drugs, the small molecule part is mainly composed of chemical toxins and chemical connectors. The commonly used chemical toxins include tubulin polymerase inhibitors, such as monomethyl auristatin D, E, F (referred to as MMAD, MMAE, MMAF), maytansine, and toxins that damage the DNA double helix structure, such as calicheamicins and duocarmycin, etc. At present, ADC drugs under research or clinical application mostly use MMAE, MMAF, tubulin depolymerizing agents maytansin DM1 and DM4, etc. The chemical connection bonds recognized at home and abroad are degradable chemical bonds such as hydrazone bonds and disulfide bonds, and non-degradable chemical bonds such as thioether bonds. In the first-generation ADC drug Mylotarg, two degradable bonds, disulfide bonds and hydrazone bonds, were used to connect the antibody to calicheamicin. Due to the unstable chemical bonds and limited therapeutic effect, it has been recalled by Pfizer in 2010. The second-generation ADC drug Kadcyla utilizes non-degradable thioether bonds and has good activity and low biological toxicity. However, due to the *in vivo* oxidation of the thioether bonds, the chemical bonds may still be broken in the blood circulation, so it may have strong liver toxicity. Since the listing of Adcetris and Kadcyla, more than 100 ADC drug candidates have entered clinical trials in humans. However, only two ADCs in China have been approved for human clinical trials, and most of the ADCs are still in the preclinical research stage. There is no fully innovative ADC antibody drug with independent intellectual property rights on the market. Therefore, the development of anti-TRAILR2 antibody-toxin-conjugates with independent intellectual property rights, good safety, and remarkable efficacy has extremely attractive clinical application prospects.

Antibodies used for preparation of ADC must have two basic characteristics, namely the tumor specificity of the antibody, and the ability of the antigen-antibody complex formed by the binding of the antibody and the antigen to be endocytosed into the lysosome and degraded in the lysosome to release small molecule toxins, which can specifically kill tumor cells.

Compared with other antibodies that have entered human clinical trials at home and abroad against the target of TRAILR2, the humanized monoclonal antibody against TRAILR2 (TRAILR2 or CD262 or DR5) with independent intellectual property rights involved in the present invention has unique gene sequences, antigenic determinants and strong antigen affinity, can specifically kill a variety of TRAILR2-positive tumor cells *in vivo* and *in vitro,* and inhibit tumor cell growth, but have little toxicity to normal cells and tissues. In the world, for the treatment of tumors with TRAILR2 as the target, the results of clinical trials show that the safety is good, but the efficacy of the drug alone is not satisfactory.

All publications mentioned herein are incorporated by reference as if each individual document was cited as a reference in the present application. It should be understood that, after reading the above teachings of the present invention, those skilled in the art can make various modifications and changes. These equivalent forms are also within the scope defined by the claims appended hereto.

### Sequence information of the present invention

The amino acid sequence of the heavy chain variable region (VH) of Zaptuzumab SEQ ID No: 7:

Gln Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Asp Phe
            20                  25                  30
Ser Met Asn Trp Val Arg Gln Ala Pro Gly Gln Gly Leu Glu Trp Met
        35                  40                  45
Gly Trp Ile Asn Thr Glu Thr Gly Glu Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Ile Asp Tyr Trp Gly Gln Gly Thr Thr Val Thr Val Ser Ser
            100                 105                 110

The amino acid sequence of the light chain variable region (VL) of the antibody SEQ ID No: 8:

Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Val His Ser
            20                  25                  30
Asn Gly Asn Thr Tyr Leu His Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Phe Gln Ser
                85                  90                  95
Thr His Val Pro His Thr Phe Gly Gln Gly Thr Lys Leu Glu Ile Lys
            100                 105                 110
Arg

ÐòÁÐ±í

<110>  °ÍÔªÉúÎï¼¼Êõ£¨ÉÏº£©¹É·ÝÓÐÏÞ¹«Ë¾

<120>  ¿¹TRAILR2¿¹Ìå-¶¾ËØ-Å¼Áª Îï¼°ÆäÔÚ¿¹Ö×ÁöÖÎÁÆÖÐµÄÒ©ÎïÓÃÍ¾

<130>  P2018-0432

<150>  2018101508705
<151>  2018-02-13

<150>  201810150871X
<151>  2018-02-13

<160>  8

<170>  PatentIn version 3.5

<210>  1
<211>  5
<212>  PRT
<213>  ÈË¹¤ÐòÁÐ(Artificial sequence)



<220>
<221>  PEPTIDE
<222>  (1)..(5)
<223>  CDRH1

<400>  1

Asp Phe Ser Met Asn
1               5



<210>  2
<211>  17
<212>  PRT
<213>  ÈË¹¤ÐòÁÐ(Artificial sequence)



<220>
<221>  PEPTIDE
<222>  (1)..(17)
<223>  CDRH2

<400>  2

Trp Ile Asn Thr Glu Thr Gly Glu Pro Thr Tyr Ala Asp Asp Phe Lys
1               5                   10                  15


Gly




<210>  3
<211>  3
<212>  PRT
<213>  ÈË¹¤ÐòÁÐ(Artificial sequence)



<220>

<221>  PEPTIDE
<222>  (1)..(3)
<223>  CDRH3

<400>  3

Ile Asp Tyr
1


<210>  4
<211>  16
<212>  PRT
<213>  ÈË¹¤ÐòÁÐ(Artificial sequence)


<220>
<221>  PEPTIDE
<222>  (1)..(16)
<223>  CDRL1

<400>  4

Arg Ser Ser Gln Ser Leu Val His Ser Asn Gly Asn Thr Tyr Leu His
1               5                   10                  15


<210>  5
<211>  7
<212>  PRT
<213>  ÈË¹¤ÐòÁÐ(Artificial sequence)


<220>
<221>  PEPTIDE
<222>  (1)..(7)
<223>  CDRL2

<400>  5

Lys Val Ser Asn Arg Phe Ser
1               5


<210>  6
<211>  9
<212>  PRT
<213>  ÈË¹¤ÐòÁÐ(Artificial sequence)


<220>
<221>  PEPTIDE
<222>  (1)..(9)
<223>  CDRL3

<400>  6

Phe Gln Ser Thr His Val Pro His Thr
1               5


<210>  7
<211>  112

<212>  PRT
<213>  ÈË¹¤ÐòÁÐ(Artificial sequence)

<220>
<221>  PEPTIDE
<222>  (1)..(112)
<223>  VH

<400>  7

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asp Phe
            20                  25                  30

Ser Met Asn Trp Val Arg Gln Ala Pro Gly Gln Gly Leu Glu Trp Met
        35                  40                  45

Gly Trp Ile Asn Thr Glu Thr Gly Glu Pro Thr Tyr Ala Asp Asp Phe
    50                  55                  60

Lys Gly Arg Phe Val Phe Ser Leu Asp Thr Ser Val Ser Thr Ala Tyr
65                  70                  75                  80

Leu Gln Ile Ser Ser Leu Lys Ala Glu Asp Thr Ala Val Tyr Tyr Cys
                85                  90                  95

Ala Arg Ile Asp Tyr Trp Gly Gln Gly Thr Thr Val Thr Val Ser Ser
            100                 105                 110

<210>  8
<211>  113
<212>  PRT
<213>  ÈË¹¤ÐòÁÐ(Artificial sequence)

<220>
<221>  PEPTIDE
<222>  (1)..(113)
<223>  VL

<400>  8

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Ser
                85                  90                  95

Thr His Val Pro His Thr Phe Gly Gln Gly Thr Lys Leu Glu Ile Lys
            100                 105                 110

Arg

## Claims

1. an antibody-drug conjugate or the pharmaceutically acceptable salt or solvent compound thereof, wherein the antibody-drug conjugate comprises:
(a) an antibody moiety; and
(b) a coupling moiety coupled to the antibody moiety, which is selected from the group consisting of a detectable label, a drug, a toxin, a cytokine, a radionuclide, an enzyme, and a combination thereof;
wherein, the heavy chain variable region of the antibody comprises the following three complementary determining regions CDRs:
(H1) CDRH1, shown in SEQ ID NO: 1,
(H2) CDRH2, shown in SEQ ID NO: 2, and
(H3) CDRH3, shown in SEQ ID NO: 3;
wherein, any one of the amino acid sequences in the above heavy chain variable region amino acid sequence also includes a derivative sequence optionally added, deleted, modified, and/or substituted with at least one amino acid, and capable of retaining the binding affinity of TRAILR2; and/or
the light chain variable region of the antibody comprises the following three complementary determining regions CDRs:
(L1) CDRL1, shown in SEQ ID NO: 4,
(L2) CDRL2, shown in SEQ ID NO: 5, and
(L3) CDRL3, shown in SEQ ID NO: 6;
wherein, any one of the amino acid sequences in the above light chain variable region amino acid sequence also includes a derivative sequence optionally added, deleted, modified, and/or substituted with at least one amino acid, and capable of retaining the binding affinity of TRAILR2.

2. The antibody-drug conjugate or the pharmaceutically acceptable salt or solvent compound thereof according to claim 1, wherein the antibody-drug conjugate ADC is shown as the following molecular formula: 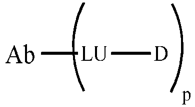 wherein,
Ab is an anti-TRAILR2 antibody;
LU is none or a linker connecting the antibody and the drug;
D is a drug;
p is the number of the drugs conjugated to the antibody; p is a value selected from 1-10, preferably 1-8, more preferably 2-4;
"-" is a bond or a linker.

3. The antibody-drug conjugate or the pharmaceutically acceptable salt or solvent compound thereof according to claim 2, wherein the structure of the LU is shown as the following fomula (I):
-L₁-L₂-L₃- (I)
wherein,
L₁ is none or Py, Mc;
L₂ is none or Vc, MAA, Mc;
L₃ is none or PAB, MAA, Mc;
each "-" is independently a key;
Py is 1,1',1"-(1,3,5-triazine-1,3,5-triyl)tri(prop-2-en-1-one);
Vc is (S)-2-((S)-2-amino-3-methylbutyrylamino)-5-ureidovaleramide;
Mc is 6-(2,5-dioxocyclopent-3-en-1-yl) hexanoic acid;
PAB-OH is (4-aminophenyl) methanol;
MAA is 2-mercaptoacetic acid,
wherein the molecular structure is as follows: 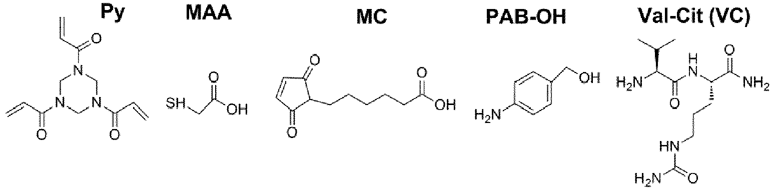 and at least one of L₁, L₂, L₃ is other than absent.

4. The antibody-drug conjugate or the pharmaceutically acceptable salt or solvent compound thereof according to claim 2, wherein the D is selected from the group consisting of: a chemotherapeutic agent, a radioactive substance, a toxin, an anti-cancer prodrug activating enzyme capable of converting the anti-cancer prodrugs into the active forms thereof, and a combination thereof.

5. The antibody-drug conjugate or the pharmaceutically acceptable salt or solvent compound thereof according to claim 2, wherein the drug D is selected from the following group: monomethyl auristatin F (MMAF), monomethyl auristatin E (MMAE), monomethyl auristatin D (MMAD), derivatives thereof, and a combination thereof.
| | | |
|---|---|---|
| D1 | monomethyl auristatin F (MMAF) | 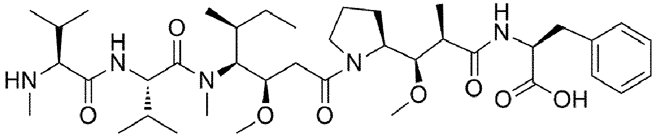 |
| D2 | monomethyl aulistatin E (MMAE) | 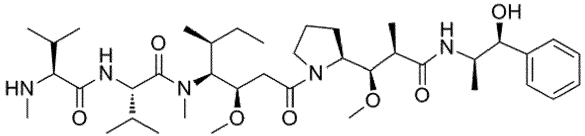 |
| D3 | monomethyl aulistatin **D** (MMAD) | 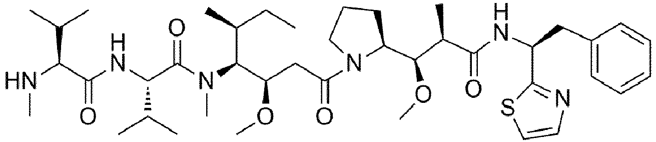 |

6. The antibody-drug conjugate or the pharmaceutically acceptable salt or solvent compound thereof according to claim 1, wherein the antibody-drug conjugate (ADC) is selected from the group consisting of: Zapadcine-1a, Zapadcine-1b, Zapadcine-1c, Zapadcine-1d, Zapadcine-3a, Zapadcine-3b, Zapadcine-3c, Zapadcine-3d, Zapadcine-3e; wherein,
the structure of conjugate Zapadcine-1a is as follows: 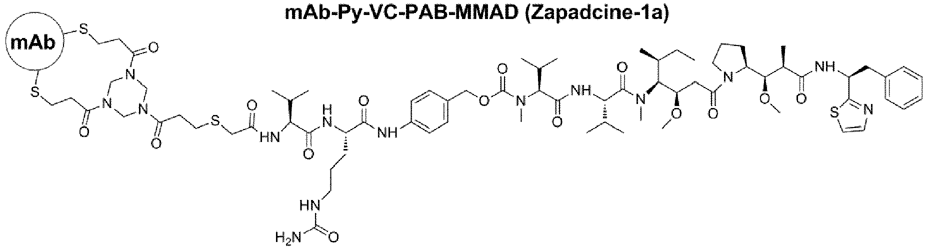 the structure of conjugate Zapadcine-1b is as follows: 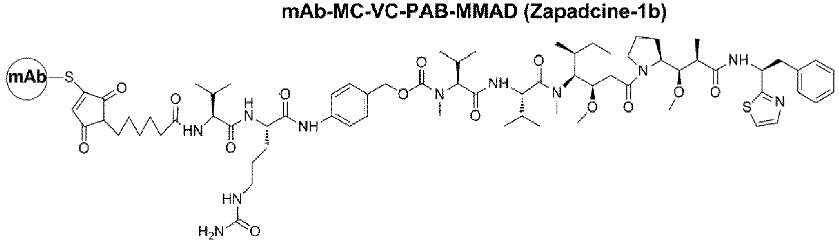 the structure of conjugate Zapadcine-1c is as follows: 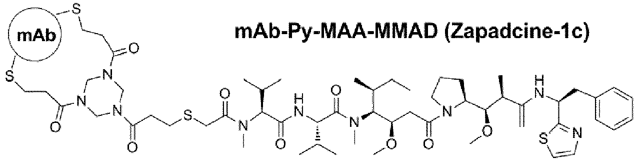 the structure of conjugate Zapadcine-1d is as follows: 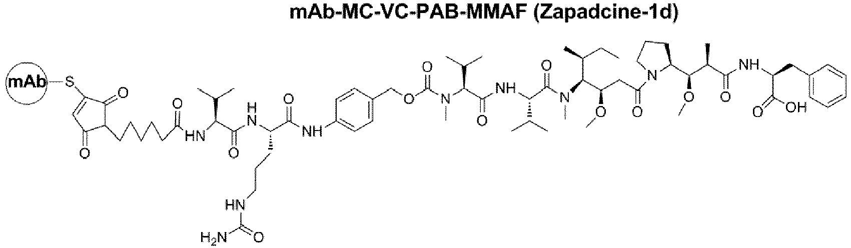 the structure of conjugate Zapadcine-3a is as follows: 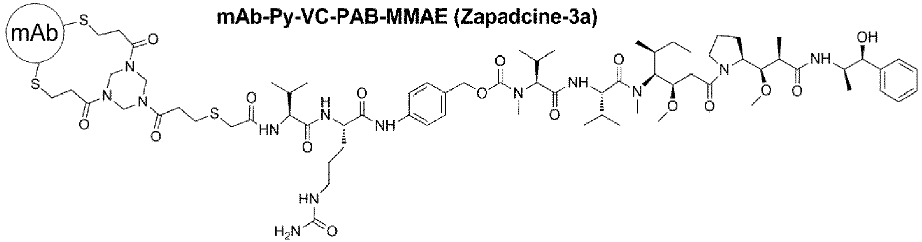 the structure of conjugate Zapadcine-3b is as follows: 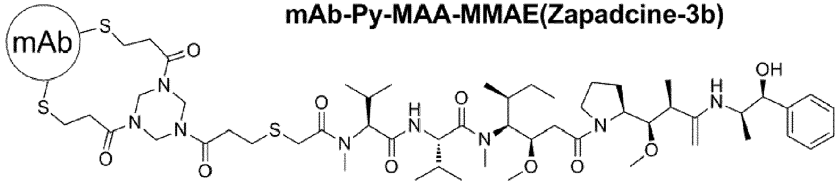 the structure of conjugate Zapadcine-3c is as follows: 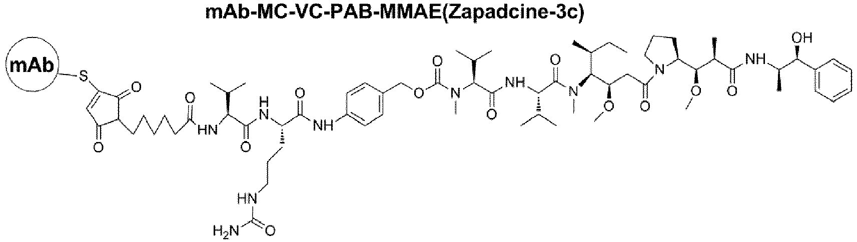 the structure of conjugate Zapadcine-3d is as follows: 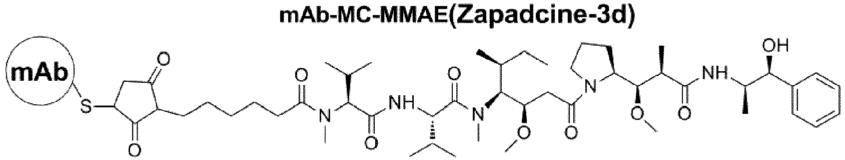 the structure of conjugate Zapadcine-3e is as follows:

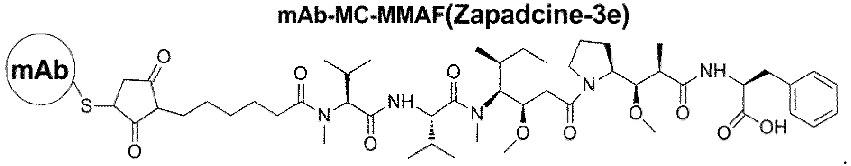

7. The antibody-drug conjugate or the pharmaceutically acceptable salt or solvent compound thereof according to any one of claims 1-6, wherein the amino acid sequence of the heavy chain variable region of the antibody is shown in SEQ ID No: 7; and/or, the amino acid sequence of the light chain variable region of the antibody is shown in SEQ ID No: 8;
wherein, the amino acid sequence of the heavy chain variable region further includes a derivative sequence with at least one amino acid optionally added, deleted, modified, and/or substituted, having a homology or sequence identity of at least 80% with the amino acid sequence shown in SEQ ID No: 7;
wherein, the amino acid sequence of the light chain variable region further includes a derivative sequence with at least one amino acid optionally added, deleted, modified, and/or substituted, having a homology or sequence identity of at least 80% with the amino acid sequence shown in SEQ ID No: 8.

8. The antibody-drug conjugate or the pharmaceutically acceptable salt or solvent compound thereof according to claim 7, wherein the antibody-drug conjugate (ADC) is selected from the group consisting of: Zapadcine-3a, Zapadcine-3d; wherein,
the structure of conjugate Zapadcine-3a is as follows: 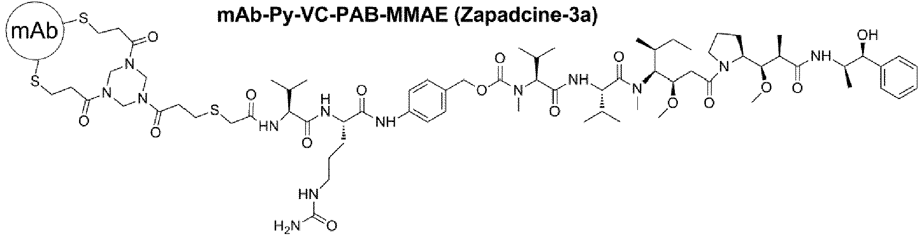 the structure of conjugate Zapadcine-3d is as follows:

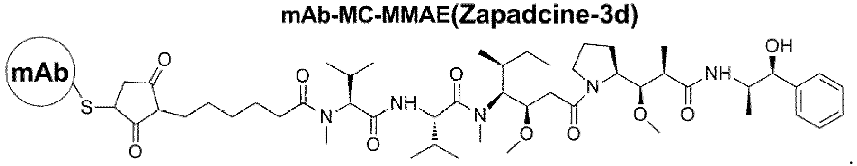

9. The antibody-drug conjugate or the pharmaceutically acceptable salt or solvent compound thereof according to claim 8, wherein the antibody-drug conjugate (ADC) is Zapadcine-3a, wherein,
the structure of conjugate Zapadcine-3a is as follows: 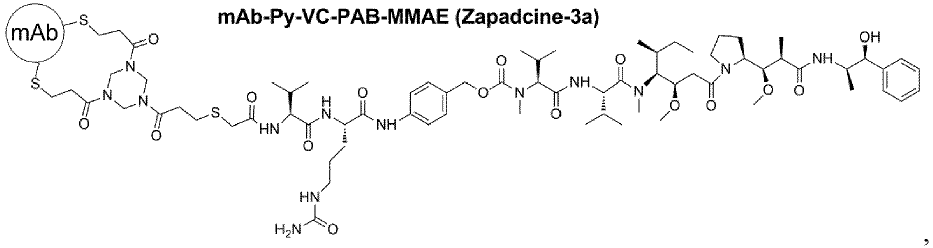 wherein, the monoclonal antibody (mAb) is Zaptuzumab.

10. Use of the antibody-drug conjugate or the pharmaceutically acceptable salt or solvent compound thereof according to claim 1, for (i) preparation of a diagnostic reagent ; and/or (ii) preparation of a medicament for the prevention and/or treatment of TRAILR2-related diseases.

11. The use according to claim 10, wherein the TRAILR2-related disease is selected from the group consisting of tumorigenesis, tumor growth and/or metastasis.

12. The use according to claim 11, wherein the cancer is a TRAILR2 positively expressing cancer and the cancer is selected from: T lymphocytic leukemia, B lymphocytic leukemia, non-T and non-B lymphocytic leukemia, non-small cell lung cancer, liver cancer, colon cancer, breast cancer, ovarian cancer, pancreatic cancer, thyroid cancer, head and neck squamous cell carcinoma, esophageal squamous cell carcinoma, lung squamous cell carcinoma, lung adenocarcinoma, cervical squamous cell carcinoma, pancreatic squamous cell carcinoma, colon squamous cell carcinoma, gastric squamous cell carcinoma, prostate cancer, osteosarcoma and soft tissue sarcoma.

13. A pharmaceutical composition, comprising:
(i) an active ingredient, which is the antibody-drug conjugate or the pharmaceutically acceptable salt or solvent compound thereof according claim 1, or a combination thereof; and
(ii) a pharmaceutically acceptable carrier.

14. The pharmaceutical composition according to claim 13, wherein the active ingredient is the antibody-drug conjugate or the pharmaceutically acceptable salt or solvent compound thereof according to claim 7 or 8 or 9, or a combination thereof.

15. An *in vitro* non-therapeutic method for inhibiting tumor cells, comprising the steps of: contacting the tumor cells with the antibody-drug conjugate or the pharmaceutically acceptable salt or solvent compound thereof according to claim 1 or 7 or 8 or 9.

16. A method for preventing and/or treating tumors, comprising the steps of: administering to the subject in need the antibody-drug conjugate or the pharmaceutically acceptable salt or solvent compound thereof according to claim 1 or 7 or 8 or 9, or the pharmaceutical composition according to claim 13 or 14.

17. Use of the antibody-drug conjugate or the pharmaceutically acceptable salt or solvent compound thereof according to claim 1 or 7 or 8 or 9, or the pharmaceutical composition according to claim 13 or 14, in the preparation of a medicament for preventing and/or treating tumors.
